(19) Europäisches Patentamt
European Patent Office
Office européen des brevets

(11) EP 1 224 270 B1

# (12) EUROPEAN PATENT SPECIFICATION

(45) Date of publication and mention of the grant of the patent:
**14.09.2005 Bulletin 2005/37**

(21) Application number: **99963846.3**

(22) Date of filing: **29.10.1999**

(51) Int Cl.[7]: **C12N 9/42**, C11D 3/386, D06M 16/00

(86) International application number:
**PCT/US1999/025394**

(87) International publication number:
**WO 2001/032848 (10.05.2001 Gazette 2001/19)**

## (54) MIMIC CELLULOSE BINDING DOMAIN

IMITATOR-ZELLULOSEBINDUNGSDOMÄNE

DOMAINE MIMETIQUE DE LIAISON A LA CELLULOSE

(84) Designated Contracting States:
**AT BE CH CY DE DK ES FI FR GB GR IE IT LI LU MC NL PT SE**

(43) Date of publication of application:
**24.07.2002 Bulletin 2002/30**

(73) Proprietor: **THE PROCTER & GAMBLE COMPANY**
**Cincinnati, Ohio 45202 (US)**

(72) Inventors:
- **BJORKQUIST, Dave, William**
  **Wyoming, OH 45215 (US)**
- **SMETS, Johan**
  **B-3210 Lubbeek (BE)**
- **SIEGEL, David, Paul**
  **Columbus, OH 43220 (US)**
- **BOYER, Stanton, Lane**
  **Fairfield, OH 45014 (US)**

(74) Representative:
**Morelle, Evelyne Charlotte Isabelle et al**
**N.V. Procter & Gamble Services Company S.A.**
**Temselaan 100**
**1853 Strombeek-Bever (BE)**

(56) References cited:
**WO-A-00/18898** **WO-A-97/28243**
**WO-A-98/00500**

- **TOMME P ET AL: "Characterization and affinity applications of cellulose-binding domains" JOURNAL OF CHROMATOGRAPHY B: BIOMEDICAL SCIENCES & APPLICATIONS,NL,ELSEVIER SCIENCE PUBLISHERS, vol. 715, no. 1, 11 September 1998 (1998-09-11), pages 283-296, XP004147002 ISSN: 0378-4347**
- **ONG E ET AL: "THE CELLULOSE-BINDING DOMAINS OF CELLULASES: TOOLS FOR BIOTECHNOLOGY" TRENDS IN BIOTECHNOLOGY,GB,ELSEVIER PUBLICATIONS, CAMBRIDGE, vol. 7, no. 9, 1 September 1989 (1989-09-01), pages 239-243, XP000604607 ISSN: 0167-7799**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

EP 1 224 270 B1

## Description

### Field of the Invention

The present invention relates to a mimic cellulose binding domain and to a chemical entity wherein the chemical entity comprises a benefit agent attached to a mimic cellulose binding domain. The present invention further relates to laundry detergent and/or fabric care compositions comprising such mimic cellulose binding domain and/or such chemical entity.

### Background of the invention

Modern laundry detergent and/or fabric care compositions contain various detergent ingredients having one or more purposes in obtaining fabrics which are not only clean, fresh and sanitised but also have retained appearance and integrity. Therefore, detergent components such as perfumes, soil release agents, fabric brightening agents, fabric softeners, chelants, bleaching agents and catalysts, dye fixatives and enzymes, have been incorporated in laundry detergent and/or fabric care compositions. In using such detergent components, it is important that some of these compounds deposit on the fabrics so as to be effective during or after the laundering and/or fabric care process.

One specific example is the use of cellulase enzymes for the treatment of cotton-containing fabrics which largely consist of cellulose. The cellulose structures are depolymerized or cleaved by cellulases into smaller and thereby more soluble or dispersible fractions. This activity in particular on fabrics provides a cleaning, rejuvenation, softening and generally improved handfeel characteristics to the fabric structure. However, it has been difficult to incorporate cellulase enzymes into modern detergents in an effective manner. In that regard, those skilled in the art have sought to use minimal amounts of cellulase to their fullest effectiveness by ensuring that most, if not all, of the cellulase enzyme comprised in the detergent composition deposits on the fabric. For example, optimum cellulases would generally have a binding domain especially suitable for celluloses. In this way, most of the cellulase enzyme included in the detergent composition deposits or otherwise binds to the fabric during the laundering cycle to achieve its desired results.

Similarly, laundry detergent and/or fabric care compositions have been formulated in which benefit agents are also modified to ensure deposition onto the fabrics :
Enzymes linked to Cellulose Binding Domains are described in the art : WO 91/10732 describes novel derivatives of cellulase enzymes combining a core region derived from an endoglucanase producible by a strain of *Bacillus* spp., NICMB 40250 with a CBD derived from another cellulase enzyme, or combining a core region derived from another cellulase enzyme with a CBD derived from said endoglucanase, for improved binding properties. WO94/07998 describes cellulase variants of a cellulase classified in family 45, comprising a CBD, a Catalytically Active Domain (CAD) and a region linking the CBD to the CAD, wherein one or more amino acid residues have been added, deleted or substituted and /or another CBD is added at the opposite end of the CAD. WO95/16782 relates to the cloning and high level expression of novel truncated cellulase proteins or derivatives thereof in *Trichoderma longibrachiatum* comprising different core regions with several CBDs. WO97/01629 describes cellulolytic enzyme preparation wherein the mobility of the cellulase component may be reduced by adsorption to an insoluble or soluble carrier e.g. via the existing or newly introduced CBD. WO97/28243 describes a process for removal or bleaching or soiling or stains from cellulosic fabrics wherein the fabric is contacted in aqueous medium with a modified enzyme which comprises a catalytically active amino acid sequence of a non-cellulolytic enzyme selected from amylases, proteases, lipases, pectinases and oxidoreductases, linked to an amino acid sequence comprising a cellulose binding domain and a detergent composition comprising such modified enzyme and a surfactant. WO98/00500 discloses a composition comprising a deposition aid having a high affinity for fibres or a surface and having a benefit agent attached / adsorbed thereto.

However, there always remains a need to formulate laundry detergent and/or fabric care compositions wherein the chemical components have improved deposition on fabrics, which results in improved performance during typical washing / fabric care cycles. There is also a need to ensure that chemical components remain on the fabric or fibre during the additional steps of rinsing and during wear. There also remains a need for such components to be formulated in a cost-effective manner.

The above objectives have been met by designing a mimic cellulose binding domain also referred to as Mimic CBD and by formulating a chemical entity wherein a benefit agent is attached to such Mimic CBD. The invention further provides a laundry detergent and/or fabric care composition comprising such chemical entity. It has been surprisingly found that such Mimic CBD binds much more effectively to cellulose than natural CBDs. Therefore, chemical entities wherein a benefit agent is attached to such a Mimic CBD more readily affix or otherwise come into contact with the fabric, thereby resulting in new, increased, enhanced and/or more cost-effective performance of the chemical component at the fabric surface or when released during or after the laundry and/or fabric care process.

## Summary of the invention

The present invention relates to a mimic cellulose binding domain and to a chemical entity wherein the chemical entity comprises a benefit agent attached to a mimic cellulose binding domain, for improved deposition onto the fabric. The present invention further relates to laundry detergent and/or fabric care compositions comprising such chemical entity, for improved cleaning and/or fabric care performance.

The present invention is further directed to laundry detergent and/or fabric care compositions comprising one or more Mimic CBD(s), linked together or not, for fabric care.

## Detailed description of the invention

The present invention relates to a mimic cellulose binding domain (hereinafter referred to as "Mimic CBD") and to a chemical entity wherein the chemical entity comprises a benefit agent attached to a Mimic CBD.

It has surprisingly been found that the mimic cellulose binding domain of the present invention binds much more effectively to cellulose than natural cellulose binding domain on all types of celluloses : crystalline, amorphous or Avicel. Therefore, the chemical entity of the present invention wherein a benefit agent is attached to such a Mimic CBD can be delivered to the fabric surface at a much higher efficiency in molecular weight of the carrier CBD and in price. Hence, laundry detergent and/or fabric care compositions comprising the chemical entity of the present invention, demonstrate improved fabric care performance including anti-wrinkling, anti-bobbling and anti-shrinkage properties, as well as fabric softness and/or improved cleaning performance, including soil and stain removal, "dingy" fabric cleaning, and whiteness maintenance.

Moreover, these Mimic CBDs are formulated with only the amino acids necessary for an effective binding to the cellulose and/or for the binding of the benefit agent. Hence, these Mimic CBDs have a higher molecular weight efficiency and are able to deliver the same amount of benefit agent than a natural CBD but at a significantly lower molecular weight. Indeed, natural CBDs have a molecular weight of about 5 to 40kDa whereas the Mimic CBDs of the present invention have a molecular weight of about 2 kDa. Therefore, the Mimic CBDs are much more efficiently formulated and are more cost-effective.

Moreover, the amino acid sequence of the Mimic CBD can be easily tailored to each application such to suit the detergent matrix, the number/amount and nature of benefit agent.

### The mimic cellulose binding domain or Mimic CBD

The present invention is directed to a mimic cellulose binding domain or Mimic CBD. As meant therein, the term "mimic" is intended to mean "a synthetic, not natural, i.e., not found in the nature as such".

In nature, the part of a polypeptide or protein (e.g. hydrolytic enzyme) which constitutes a CBD per se typically consists of more than about 30 and less than about 250 amino acid residues. For example, those CBDs listed and classified in Family I in accordance with P. Tomme et al. (op. cit.) consist of 33-37 amino acid residues, those listed and classified in Family IIa consist of 95-108 amino acid residues, those listed and classified in Family VI consist of 85-92 amino acid residues, whilst one CBD (derived from a cellulase from Clostridium thermocellum) listed and classified in Family VII consists of 240 amino acid residues. Accordingly, the molecular weight of an amino acid sequence constituting a CBD per se will typically be in the range of from about 4kD to about 40kD, and usually below about 35kD.

For the purposes of the present invention, the mimic cellulose binding domain is an amino acid sequence capable of effective binding to a cellulosic substrate. The Mimic CBD of the present invention is an amino acid sequence of less than 30 amino acids with high affinity for cellulose structures, i.e. which has a protein deposition of more than 50% on the cellulose structure (Avicel PH105) at a 1micromolar concentration. Preferably, the Mimic CBD of the present invention has a protein deposition of more than 75% on the cellulose structure (Avicel PH105) at a 1 micromolar concentration.

Such protein deposition is defined as follows :
The binding assay was typically performed by adding a solution of the Mimic CBD to 0.25 g of Avicel (PH105) and stirring overnight at 70°C. After the equilibration period, the Mimic CBD-Avicel slurry was centrifuged and the supernatant removed for fluorescent measurement. The extent of binding was determined by the difference in fluorescence between an experiment performed in the presence and absence of Avicel. The extent of binding is expressed as the percentage of the Mimic CBD that binds to Avicel when added at an initial concentration of 1 micromole. If the Mimic CBD contained tryptophan, no additional fluorescent marker had to be added to obtain a signal. However, for those Mimic CBDs lacking tryptophan, fluorescein was attached at the N-terminus of those Mimic CBDs.

## Materials

The Mimic CBD for the binding studies were purchased from either Midwest Biotech Inc. or Research Genetics, Inc., and were received in greater than 95% purity. The source of cellulose was Avicel PH105 (FMC Corporation).

## Binding Studies

The method for a typical assay follows.

Solutions of the Mimic CBDs were prepared by dissolving a weighed amount in the appropriate solvent (buffer or 0.01 M HCl) to approximately a concentration of 0.1 mg/ml. The exact concentration varied based on the solubility of the Mimic CBD and the amount available. Typically, 1 mg of Mimic CBDs would be weighed and diluted in 10 ml increments until the Mimic CBD was solubilized. A UV/Vis absorbance spectrum was obtained in order to determine the concentration of the sample. The concentration was determined from the absorbance at $\lambda_{max}$ for each Mimic CBD using the literature value for the molar extinction coefficient (tryptophan, 5550 $cm^{-1}M^{-1}$; fluorescein, 77000 $cm^{-1}M^{-1}$).

While the Mimic CBD was solubilizing, the cellulose substrate was prepared. 0.250 +/-0.001 g of cellulose was weighed into a series of Pyrex® tubes. Each sample was washed with the assay diluent thrice by pipetting the diluent into the tube, vortex mixing, spinning the samples in a Beckman GS-6KR centrifuge (1000 rpm) for 15 minutes at room temperature, aspirating the supernatant, and repeating the wash procedure. Typically, 2 ml of diluent were used per wash. The diluent was varied based on the solubility of the Mimic CBD and the test conditions under examination. Mimic CBDs where tryptophan served as the fluorophore were usually dissolved in 10 mM HCl, while fluorescein labeled compounds were dissolved in either 10 mM TES (N-tris[Hydroxymethyl]methyl-2-aminoethanesulfonic acid) pH 7.00 or 5 mM TES pH 7.40.

Once the cellulose samples were ready, a known amount of the Mimic CBD solution was pipeted into the tubes, which also contained a small Teflon® coated stirring bar. Each analysis was run in duplicate. Additionally, several tubes that only contained the diluent were prepared along with several tubes that contained cellulose and diluent to serve as controls. All samples were then vortex mixed and transferred to a room temperature water bath placed on a magnetic multi-place stirring plate in a constant temperature room (controlled temperature was maintained at 70 degrees C +/- 2 degrees C). The samples were stirred overnight. The following morning, the samples were removed to a Beckman GS-6KR centrifuge and spun for 15 minutes to pellet the cellulose substrate in each tube. All tubes whether they contained cellulose or not, were treated in the exact same fashion, i.e., spins, stirring bars, etc.

The supernatant of each sample was then transferred to a fresh tube via Pasteur pipet. Each of these samples were centrifuged again in order to pellet any residual cellulose debris from the transfer process. The fluorescence of each sample was then read on a Spex Fluorolog fluorometer after the excitation minimum and maximum had been determined for one of the test concentrations, typically 1 μM. In order to examine higher concentrations for binding (generally, concentrations over 2 μM were off scale), the samples were prepared as above, but were diluted in the assay diluent in order to read the fluorescence.

Once the fluorescence data were obtained, the value of the background signal (diluent only) was subtracted from each reading (diluent plus Mimic CBD) and a calibration curve generated by plotting the fluorescence cps (counts per second) data versus the concentration of Mimic CBD.

From this plot, the slope and intercept were obtained and were used to interpret the binding data. The amount of Mimic CBD not bound to cellulose, i.e. free Mimic CBD in solution, for any particular experiment was determined by measuring the fluoresence of the supernatant from that experiment; subtracting the background signal (diluent plus cellulose); and determining the concentration from the calibration curve. The amount of Mimic CBD bound was equal to the starting concentration of the Mimic CBD less the concentration of the free Mimic CBD in solution. The percent of compound bound was then calculated by dividing the bound concentration by the starting concentration and multiplying by 100.

The Mimic CBD amino acid sequence can comprise any of the natural occurring amino acids, alanine, serine, aspartic acid, arginine, valine, threonine, glutamic acid, leucine, cysteine, histidine, lysine, isoleucine, tyrosine, asparagine, methionine, proline, tryptophan, phenylalanine, glutamine, glycine and mixtures thereof. Encompassed in the present invention but in a less preferred option, some of the naturally occuring amino can be replaced by non-natural occurring amino acids like aminohexanoic acid or aminocaprylic acid. Such non-natural amino acid are less preferred since the resulting amino acid sequence is not producible via fermentation routes.

The Mimic CBD comprises in its amino acids sequence, at least 3 non-polar amino acids, like alanine, proline, valine, leucine, isoleucine, tyrosine, tryptophan and/or phenylalanine; preferably at least 3 non-polar amino acids selected from valine, leucine, isoleucine, tyrosine, tryptophan and/or phenylalanine; more preferably at least 3 aromatic amino adds like tyrosine, tryptophan and/or phenylalanine. Indeed, without wishing to be bound by theory, it is believed that the aromatic groups in the side chain of these amino acids are very efficient for the binding to the cellulose structures.

Preferably, the Mimic CBDs of the present invention will have the following structure wherein said non-polar amino acids separated by at least 1, and preferably 1, other amino acid referred to as "separating amino acid". Those separating amino acids are preferably selected from glycine, alanine, glutamine, asparagine, aspartate, glutamate, lysine, arginine or histidine. Indeed, without wishing to be bound by theory, it is believed that the H-bond centers of these amino acids provide a tighter binding of the amino acid sequence to the cellulose structure.

The amino acids sequence of the Mimic CBD comprises less than 30 amino acids, preferably less than 12, more preferably less than 10 or even less amino acids.

The Mimic CBD can further comprise, at one or each end, one or more amino acids which can be used to link the benefit agent and which are responsible for delivering the benefit agent as described below. Those amino acids will be referred to as "attachement amino acids". The attachement amino acids can be linked together to form an amino acid sequence offering several attachment points for benefit agents. The attachement amino acids are preferably selected from lysine, tyrosine, arginine, histidine, asparagine, glutamine, and/or cysteine. Therefore, the benefit agent of the chemical entity of the present invention can be attached to some amino acids within the separating amino acids or preferably, to those attachement amino acids linked at the terminus of the Mimic CBD.

The number of attachment amino acids depends on the type of benefit agent and the application. Therefore the number of the attachment amino acids can vary from 1 to 20. For instance when the benefit agent is an enzyme, only one attachment amino acid is needed for the attachment of the enzyme to the Mimic CBD.

Mimic cellulose binding domains can be produced by recombinant techniques as described in H. Stålbrand et al., Applied and Environmental Microbiology, Mar. 1995, pp. 1090-1097; E. Brun et al., (1995) Eur. J. Biochem. 231, pp. 142-148; J.B. Coutinho et al., (1992) Molecular Microbiology *6(9)*, pp. 1243-1252. When the benefit agent of the present invention is an enzyme, the resulting chemical entity can be produced via protein engineering and fermentation.

The following amino acid sequences exemplify suitable Mimic CBDs for the purpose of the present invention wherein the standard abbreviations are used: A = alanine; W = tryptophan, K = lysine; Q = glutamine; N = asparagine; E = glutamic acid; and I = isoleucine.

| Suitable Mimic CBDs of the present invention |
|---|
| $(AW)_4K_2$ |
| $(WQ)_4K_2$ |
| $(WN)_4K_2$ |
| $(WE)_4K_2$ |
| $A_4KW_4K$ |
| $(AI)_3AWK_2$ |
| $(AW)_3K_2$ |
| $(AW)_3K_2$ |
| $(WQ)_3K_2$ |
| $A_3KW_3K$ |

The most preferred Mimic CBDs for the purpose of the present invention are the $(AW)_4K_2$ and $(WE)_4K_2$ amino acid sequences.

Softening

Also contemplated in the present invention is the use of one or more Mimic CBD(s) per se, attached together or not, for fabric care. Encompassed in the present invention are cross-linked Mimic CBDs, i.e. amino acid sequences comprising mimic cellulose binding domains which are cross-linked to each other; and/or Mimic CBDs further linked to a softening protein, i.e. an amino acid sequence comprising a mimic cellulose binding domain linked to a softening protein. When the Mimic CBDs of the present invention are used to provide fabric care, such Mimic CBD can encompass only the amino acids responsible for the binding to the cellulose structure. The attachement amino acids responsible for delivering the benefit agent are not necessary but not excluded. Furthermore, the cross-linked Mimic CBD of the present invention can further comprise natural cellulose binding domains.

It has been surprisingly found that the laundry detergent and/or fabric care compositions of the present invention comprising one or more Mimic CBDs, provide softening, abrasion resistance and pilling prevention. Indeed, without wishing to be bound by theory, it is believed that the Mimic CBDs adsorb very effectively on the fibres of the fabric. Such adsorbed Mimic CBDs protect the fabric's fibers and thereby prevent the fibrillation of the cellulosic fibres. Moreover, Mimic CBDs are proteins and therefore provide softening per se.

Encompassed further in the present invention are cross-linked Mimic CBDs. It has been surprisingly found that such cross-linked Mimic CBDs provide very efficient softness, prevent the fibrillation of cellulosic fibers as indicated above and also restore tensile strength, prevent the apparition of wrinkles, and increase the hydrophilicity of synthetic fibers. Indeed, without wishing to be bound by theory, it is believed that each cross-linked Mimic CBD can adsorb at opposite sites of the damaged fibers and thereby can restore tensile strength. Those cross-linked Mimic CBD entity are preferably formed by the linking of 2 to 50, more preferably 2 to 10 Mimic CBDs. They can comprise as well a mixture of Mimic CBDs and natural CBDs. These cross-linked mimic cellulose binding domain entities are generally comprised withitn the compositions of the present invention at a level of from 0.001 % to 50%, preferably from 0.01 % to 20%, more preferably from 0.1 % to 10% by weight of the total composition.

A further embodiment of the present invention is the above described embodiments wherein the Mimic CBD or the crossed-linked Mimic CBD entity is further linked to a softening protein. Without wishing to be bound by theory, it is believed that the addition of a Mimic CBD to a softening protein, allows a higher concentration of the softening protein onto the fabric, i.e. a more effective, closer and/or more lasting contact, resulting in a more efficient activity. Such modified softening proteins have an increased affinity (relative to unmodified softening protein) for binding to a cellulosic fabric or textile.

The Chemical entity

The present invention is further directed to a chemical entity wherein one or more benefit agent(s) is attached - optionally via one or more linking region(s) - to one or more Mimic CBD(s).

When a benefit agent is linked to the Mimic CBD, attention should preferably be paid to the weight ratio of the benefit agent to the Mimic CBD. Preferably, the weight ratio of the benefit agent to the Mimic CBD amino acid sequence is of at least 0.1, preferably at least 0.3.

The chemical entities of the present invention are generally comprised in the laundry detergent and/or fabric care compositions at a level of from 0.00001% to 50%, preferably 0.001% to 20%, more preferably from 0.1% to 10% by weight of the total composition.

The linking region

The chemical entities of the present invention may further comprise one or more linking region(s). The term "linking region" is intended to indicate a region that adjoins the Mimic CBD and connects it to the chemical component (s) and/or that attaches one or more Mimic CBD together to form the cross-liked Mimic CBD entity.

Suitable linking regions, if encompassed in the improved chemical entity of the present invention, are characterized by having one or more attachment point(s) for a chemical component and one or more moiety that will covalently bind to the Mimic CBD. Preferably, the linking regions of the present invention will encompass at least one attachment point to the Mimic CBD and more than one reactive group available to attach a chemical component, hereinafter referred to as a "polyreactive linking region". When present, this linking region can be achieved chemically or by recombinant techniques.

Suitable linking regions for the purpose of the present invention are :

1) Suitable are the polyethylene glycol derivatives described in the Shearwater polymers, Inc. catalog of January 1996, such as the nucleophilic PEGs, the carboxyl PEGs, the electrophilically activated PEGs, the sulfhydryl-selective PEGs, the heterofunctional PEGs, the biotin PEGs, the vinyl derivatives, the PEG silanes and the PEG phospholipids. In particular, suitable non-amino acid linking regions are the heterofunctional PEG, (X-PEG-Y) polymers from Shearwater such as PEG(NPC)2, PEG-(NH2)2, t-BOC-NH-PEG-NH2, t-BOC-NH-PEG-CO2NHS, OH-PEG-NH-tBOC, FMOC-NH-PEG-CO2NHS or $PEG(NPC)_2$ MW 3400 from Sigma.
2) Other suitable linking regions are glutaric dialdehyde 50 wt% solution in water from Aldrich, disuccinimidyl suberate (DSS) form Sigma, γ-maleimidobutyric acid N-hydroxysuccinimide ester (GMBS) from Sigma, 1-ethyl-3-(3-dimethylaminopropyl)carbodiimide hydrochloride (EDC) from Sigma and dimethyl suberimidate hydrochloride (DMS) from Sigma, 1-ethyl-3-(3-dimethylaminopropyl) carbodiimide, N-ethyl-5-phenylisoaxolium-3-sulphonate, 1-cyclohexyl-3(2morpholinoethyl) carbodide metho-p-toluene sulphonate, N-ethoxycarbonyl-2-ethoxy 1,2, dihydroquinoline or glutaraldehyde.
3) Also suitable are the crosslinkers described in the 1999/2000 Pierce Products Catalogue from the Pierce Company, under the heading "Cross linking reagents : the SMPH, SMCC, LC-SMCC compounds, and preferably the Sulfo-KMUS compound.

Preferred linking regions therefrom are PEG(NPC)2, (NH2)2-PEG, t-BOC-NH-PEG-NH2, MAL-PEG-NHS, VS-PEG-NHS polymers from Shearwater and/or the Sulfo-KMUS compound from Pierce.

Suitable polyreactive linking regions include polyacrylic acid/ maleic acid polymers, polyvinyl alcohol polymers and/or amine-containing compounds as described in copending international application PCT/US98/20491 filed internationally on September 30, 1998 by P&G, hereby incorporated by reference. Such amine-containing compounds include amino aryl derivatives, polyamines, substituted amines and amides, glucamines, dendrimers / chitosan saccharides, amine derivatives polysaccharides and peptidic polymers.

1) Poly acrylic acid/maleic acid : Suitable poly acrylic acid, maleic acid and/or mixtures thereof are those wherein one of the acid moiety is covalently bond to a NH2 group present in the amino acid sequence of the Mimic CBD. The other acid units are the reactive groups available as potential attachment groups for chemical components via their alcohol/amine groups.

2) Poly vinyl alcohol polymers. These polymers can still comprise or not the moiety containing the polymerisation initiator.

- Without the acid moiety containing initiator of polymerisation, this polymer can be linked for example, to the reactive group of aspartic or glutamic acid present in the amino acid sequence of the Mimic CBD via an esterification reaction. The poly vinyl alcohol polymer does further comprise hydroxyl reactive groups available for attachment of acid/aldehyde containing chemical components.
- With an acid moiety containing initiator of polymerisation, this acid moiety of the initiator of polymerisation of the polymer for example, can be linked for example, to a NH2 group present in the amino acid sequence of the Mimic CBD. The poly vinyl alcohol polymer does further comprise hydroxyl reactive groups available for attachment of acid/aldehyde containing chemical components.

3) Also suitable are amine-containing compounds as described in co-pending international application PCT/US98/20491 filed internationally on September 30, 1998 by P&G, hereby incorporated by reference and having the following general structure : B-(NH2)n; wherein B is a carrier material, and n is an index of value of at least 1. Preferred B carriers are inorganic or organic carriers. By "inorganic carrier", it is meant carrier which are non-or substantially non carbon based backbones.

Preferred primary amines, among the inorganic carriers, are those selected from mono or polymers or organic-organosilicon copolymers of amino derivatised organo silane, siloxane, silazane, alumane, aluminum siloxane, or aluminum silicate compounds. Typical examples of such carriers are: organosiloxanes with at least one primary amine moiety like the diaminoalkylsiloxane [H2NCH2(CH3) 2Si]O, or the organoaminosilane (C6H5) 3SiNH2 described in: Chemistry and Technology of Silicone, W. Noll, Academic Press Inc. 1998, London, pp 209, 106).

Preferred primary amines, among the organic carriers, are those selected from aminoaryl derivatives, polyamines, amino acids and derivatives thereof, substituted amines and amides, glucamines, dendrimers, the polyvinylamines with a MW of from 600-50K; amino substituted polyvinylalcohol with a MW ranging from 400-300,000; polyoxyethylene bis [amine]; polyoxyethylene bis [6-aminohexyl]; N,N'-bis-(3-aminopropyl)-1,3-propanediamine linear or branched; 1,4-bis-(3-aminopropyl) piperazine, and mixtures thereof.

Preferred aminoaryl derivatives are the amino-benzene derivatives including the alkyl esters of 4-amino benzoate compounds, and more preferably selected from ethyl-4-amino benzoate, phenylethyl-4-aminobenzoate, phenyl-4-aminobenzoate, 4-amino-N'-(3-aminopropyl)-benzamide, and mixtures thereof.

Polyamines suitable for use in the present invention are polyethyleneimines polymers, poly[oxy(methyl-1,2-ethanediyl)], -(2-aminomethylethyl)-(2-aminomethyl-ethoxy)- (= C.A.S No. 9046-10-0); poly[oxy(methyl-1,2-ethanediyl)], -hydro-)--(2-aminomethylethoxy)-, ether with 2-ethyl-2-(hydroxymethyl)-1,3-propanediol (= C.A.S. No. 39423-51-3); commercially available under the tradename Jeffamines T-403, D-230, D-400, D-2000; 2,2',2"-triaminotriethylamine; 2,2'-diamino-diethylamine; 3,3'-diamino-dipropylamine, 1,3 bis aminoethyl-cyclohexane commercially available from Mitsibushi and the C12 Stemamines commercially available from Clariant like the C12 Sternamin(propylenamine)n with n=3/4, and mixtures thereof. Preferred polyamines are polyethyleneimines commercially available under the tradename Lupasol like Lupasol FG, G20,wfv, PR8515, WF, FC, G20, G35, G100, HF, P, PS, SK, SNA.

Preferred substituted amines and amides for use herein are selected from nipecotamide, N-coco-1,3-propenediamine; N-oleyl-1,3-propenediamine; N-(tallow alkyl)-1,3-propenediamine; 1,4-diamino cyclohexane; 1,2-diaminocyclohexane; 1,12-diaminododecane, and mixtures thereof.

Other primary amine compounds suitable for use herein are the glucamines, preferably selected from 2,3,4,5,6-pentamethoxy-glucamine; 6-acetylglucamine, glucamine, and mixture thereof.

Also preferred compounds are the polyethylenimine and/or polypropylenimine dendrimers and the commercially available Starburst polyamidoamines (PAMAM) dendrimers, generation G0-G10 from Dendritech and the dendrimers Astromols, generation 1-5 from DSM being DiAminoButane PolyAmine DAB (PA)x dendrimers with x = 2nx4 and n being generally comprised between 0 and 4.

Still other preferred primary amine compounds are :

- polyvinylamines with a molecular weight (MW) ranging from 600, 1200, 3K, 20K, 25K or 50K;
- amino substituted polyvinylalcohol with a MW ranging from 400-300,000;
- polyoxyethylene bis [amine] available from e.g. Sigma;
- polyoxyethylene bis [6-aminohexyl] available from e.g. Sigma;
- N,N'-bis-(3-aminopropyl)-1,3-propanediamine linear or branched (TPTA)
- 1,4-bis-(3-aminopropyl) piperazine (BNPP).

The more preferred compounds are ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; glucamine; the diaminobutane dendrimers Astramol, polyvinylamines with a MW ranging from 600, 1200, 3K, 20K, 25K or 50K; amino substituted polyvinylalcohol with a MW ranging from 400-300,000; polyoxyethylene bis [amine]; polyoxyethylene bis [6-aminohexyl]; N,N'-bis-(3-aminopropyl)-1,3-propanediamine linear or branched; 1,4-bis-(3-aminopropyl) piperazine, and mixture thereof. Most preferred primary amine compounds are selected from ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; the diaminobutane dendrimers Astramol, N,N'-bis-(3-aminopropyl)-1,3-propanediamine linear or branched; 1,4-bis-(3-aminopropyl) piperazine, and mixtures thereof. Even most preferred compounds are those selected from ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; N,N'-bis-(3-aminopropyl)-1,3-propanediamine linear or branched, 1,4-bis-(3-aminopropyl) piperazine, and mixtures thereof.

Preferred linking regions within the above described compounds are the polymers selected from PEG(NPC) 2, (NH2)2-PEG, t-BOC-NH-PEG-NH2, MAL-PEG-NHS VS-PEG-NHS, polyethyleneimine polymers, polyvinvyl alcohol polymers, polyamines and/or mixtures thereof.

Most preferred polyreactive linking regions for use in the present invention are amino acids and their derivatives, especially ester and amide derivatives. These peptidic polymers are linked to the Mimic CBD via a peptidic linkage. More preferred compounds are those providing enhanced surface substantivity due to its structural feature. Suitable amino acids have the following functionality of formula:

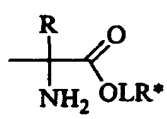

R=H, (L)-R*,

Suitable amino acids for use herein are selected tyrosine, tryptophane, lysine, glutamic acid, glutamine, aspartic acid, arginine, asparagine, phenylalanine, proline, glycine, serine, histidine, threonine, methionine, and mixture thereof. Homopolymers with the same amino acids or heteropolymers with different amino acids are suitable. For example, the amino acids Serine, Threonine and Tyrosine possess the reactive hydroxyl group, the Cysteine present a reactive SH group, the Asparagine and Glutamine amino acids possess a reactive amido group and the Lysine a reactive amino group. The linking will preferably be achieved on the Tyrosine, Cysteine or Lysine. Especially, the free NH2 group from a Lysine amino acid or of the terminal amino acid within the peptidic polymer is used as attachment points for chemicals containing aldehyde, ene one, ketone, or acid or halogene moeities

Also suitable compound are the amino acid derivatives selected from tyrosine ethylate, glycine methylate, tryptophane ethylate and mixture thereof,

These peptidic polymers can be attached to the Mimic CBD, by recombinant technology. An example of the recombinant technique describing the expression of an enzyme with the CBD of different origin is described in S. Karita et al., (1996) Journal of Fermentation and Bioengineering, Vol. 81, No. 6, pp. 553-556. The polyreactive linking region can comprise from 1 to about 100 amino acid residues, in particular of from 2 to 40 amino acid residues, e.g. from 2 to 15 amino acid residues. It is preferred to use amino acids which are less favoured by the surrounding proteases. Indeed, any combination of amino acids can be selected to achieve maximal weight efficiency and protease stability. Suitable amino acid linking regions are the *Humicola insolens* family 45 cellulase linker, the NifA gene of *Klebsiella pneumoniae*-CiP linker, the *E. coli* OmpA gene-CiP linker, the E3 cellulase *Thermomonospora fusca* linker and the CenA cellulase linker; preferably the *Humicola insolens* family 45 cellulase linker and the E3 cellulase *Thermomonospora fusca* linker.

Depending on the intended activity of the attached chemical component, said chemical components can be linked permanently or temporarily to the Mimic CBD and/or linking region. Therefore, the present invention further encompasses chemical entities wherein the chemical component is linked to the Mimic CBD and/or linking region by a weak bond. Said weak bond is a bond which can be enzymatically cleaved, oxidized, cleaved by light radiation and/or hydrolysed during or after the wash/fabric care process in order to release the chemical component(s). Examples of weak bonds are Shiff-base or beta-amino-ketone bonds.

The benefit agents

The chemical entity of the present invention comprises a benefit agent. The benefit agent is understood within the context of the present invention as any compound which gives a desirable effect on a fibre or fabric. The benefit agents encompassed in the chemical entity are preferably selected from perfumes, hygiene agents, insect control agent, enzymes, softening protein, fabric softening agents, soil release agents, bleaching agents, dye fixatives agents, brighteners, surfactants and/or mixtures thereof.

More preferred benefit agents are the perfume agents, insect control agents, hygiene agents and/or bleaching agents.

In addition to these chemical entities, the compositions of the present invention can comprise the same benefit agents unmodified.

The benefit agent of the present invention may be encapsulated. Suitable encapsulating material includes starches, poly(vinylacetate), urea/formaldehyde condensate based materials. Especially suitable encapsulating materials are water soluble capsules which consist of a matrix of polysaccharide and polyhydroxy compounds such as described in GB 1,464,616. Other suitable water soluble encapsulating materials comprise dextrins derived from ungelatinized starch acid-esters of substituted dicarboxylic acids such as described in US 3,455,838. These acid-ester dextrins are preferably, prepared from such starches as waxy maize, waxy sorghum, sago, tapioca and potato. Suitable examples of said encapsulating materials include N-Lok manufactured by National Starch. The N-Lok encapsulating material consists of a modified maize starch and glucose. The starch is modified by adding monofunctional substituted groups such as octenyl succinic acid anhydride.

***Enzymes***

A chemical entity suitable for the compositions of the present invention is a enzyme entity wherein at least one benefit agent is an enzyme linked to a Mimic CBD. It is believed that such enzyme entity results in a higher effective concentration of the enzyme at its substrate location and therefore, increased or enhanced enzymatic benefits.

Without wishing to be bound by theory, it has been surprisingly found that said enzyme entity more readily attaches, affixes or otherwise comes into closer and/or more lasting contact with the fabric, thereby resulting in increased or enhanced performance of the enzyme. In particular, the laundry detergent and/or fabric care compositions of the present invention provide improved enzymatic benefits, i.e. improved enzymatic stain removal, enhanced enzymatic fabric care and/or improved enzymatic sanitisation benefits. Said enhanced enzymatic benefits are achieved by means of a process wherein the fabric is contacted with an enzyme which has been modified so as to have increased affinity (relative to unmodified enzyme and relative to an enzyme entity wherein the enzyme has been attached to a natural CBD) for binding to a cellulosic fabric or textile.

Suitable enzymes for the purpose of the present invention are selected from cellulases, hemicellulases, peroxidases, proteases, gluco-amylases, amylases, xylanases, lipases, phospholipases, esterases, cutinases, pectinases, keratanases, reductases, oxidases, phenoloxidases, lipoxygenases, ligninases, pullulanases, tannases, pentosanases, malanases, $\beta$-glucanases, arabinosidases, mannanase, pectin degrading enzyme, xyloglucanase, hyaluronidase, chondroitinase, laccase or mixtures thereof.

Suitable lipase enzymes for detergent usage include those produced by microorganisms of the Pseudomonas group, such as Pseudomonas stutzeri ATCC 19.154, as disclosed in British Patent 1,372,034. Suitable lipases include those which show a positive immunological cross-reaction with the antibody of the lipase, produced by the microorganism *Pseudomonas fluorescent* IAM 1057. This lipase is available from Amano Pharmaceutical Co. Ltd., Nagoya, Japan, under the trade name Lipase P "Amano," hereinafter referred to as "Amano-P". Other suitable commercial lipases include Amano-CES, lipases ex *Chromobacter viscosum,* e.g. *Chromobacter viscosum var. lipolyticum* NRRLB 3673 from Toyo Jozo Co., Tagata, Japan; *Chromobacter viscosum* lipases from U.S. Biochemical Corp., U.S.A. and Disoynth Co., The Netherlands, and lipases ex *Pseudomonas gladioli.* Especially suitable lipases are lipases such as

M1 Lipase$^R$ and Lipomax$^R$ (Gist-Brocades) and Lipolase$^R$ and Lipolase Ultra$^R$(Novo) which have found to be very effective when used in combination with the compositions of the present invention. Also suitables are the lipolytic enzymes described in EP 258 068, WO 92/05249 and WO 95/22615 by Novo Nordisk and in WO 94/03578, WO 95/35381 and WO 96/00292 by Unilever.
Also suitable are cutinases [EC 3.1.1.50] which can be considered as a special kind of lipase, namely lipases which do not require interfacial activation. Addition of cutinases to detergent compositions have been described in e.g. WO-A-88/09367 (Genencor); WO 90/09446 (Plant Genetic System) and WO 94/14963 and WO 94/14964 (Unilever).

Suitable proteases are the subtilisins which are obtained from particular strains of *B. subtilis* and *B. licheniformis* (subtilisin BPN and BPN'). One suitable protease is obtained from a strain of *Bacillus,* having maximum activity throughout the pH range of 8-12, developed and sold as ESPERASE® by Novo Industries A/S of Denmark, hereinafter "Novo". The preparation of this enzyme and analogous enzymes is described in GB 1,243,784 to Novo. Other suitable proteases include ALCALASE®, DURAZYM® and SAVINASE® from Novo and MAXATASE®, MAXACAL®, PROPERASE® and MAXAPEM® (protein engineered Maxacal) from Gist-Brocades. Also suitable for the present invention are proteases described in patent applications EP 251 446 and WO 91/06637, protease BLAP® described in WO91/02792 and their variants described in WO 95/23221. See also a high pH protease from Bacillus sp. NCIMB 40338 described in WO 93/18140 A to Novo. Enzymatic detergents comprising protease, one or more other enzymes, and a reversible protease inhibitor are described in WO 92/03529 A to Novo. When desired, a protease having decreased adsorption and increased hydrolysis is available as described in WO 95/07791 to Procter & Gamble. A recombinant trypsin-like protease for detergents suitable herein is described in WO 94/25583 to Novo. Other suitable proteases are described in EP 516 200 by Unilever.
Proteolytic enzymes also encompass modified bacterial serine proteases, such as those described in European Patent Application Serial Number 87 303761.8, filed April 28, 1987 (particularly pages 17, 24 and 98), and which is called herein "Protease B", and in European Patent Application 199,404, Venegas, published October 29, 1986, which refers to a modified bacterial serine protealytic enzyme which is called "Protease A" herein. Suitable is what is called herein "Protease C", which is a variant of an alkaline serine protease from Bacillus in which lysine replaced arginine at position 27, tyrosine replaced valine at position 104, serine replaced asparagine at position 123, and alanine replaced threonine at position 274. Protease C is described in WO 91/06637. Genetically modified variants, particularly of Protease C, are also included herein.
A preferred protease referred to as "Protease D" is a carbonyl hydrolase variant having an amino acid sequence not found in nature, which is derived from a precursor carbonyl hydrolase by substituting a different amino acid for a plurality of amino acid residues at a position in said carbonyl hydrolase equivalent to position +76, preferably also in combination with one or more amino acid residue positions equivalent to those selected from the group consisting of +99, +101, +103, +104, +107, +123, +27, +105, +109, +126, +128, +135, +156, +166, +195, +197, +204, +206, +210, +216, +217, +218, +222, +260, +265, and/or +274 according to the numbering of *Bacillus amyloliquefaciens* subtilisin, as described in WO95/10591 and WO95/10592. The "protease D" variants have preferably the amino acid substitution set 76/103/104, more preferably the substitution set N76D/S103A/V104I. Also suitable is a carbonyl hydrolase variant of the protease described in WO95/10591, having an amino acid sequence derived by replacement of a plurality of amino acid residues replaced in the precursor enzyme corresponding to position +210 in combination with one or more of the following residues : +33, +62, +67, +76, +100, +101, +103, +104, +107, +128, +129, +130, +132, +135, +156, +158, +164, +166, +167, +170, +209, +215, +217, +218, and +222, where the numbered position corresponds to naturally-occurring subtilisin from *Bacillus amyloliquefaciens* or to equivalent amino acid residues in other carbonyl hydrolases or subtilisins, such as *Bacillus lentus* subtilisin (co-pending patent application published under WO98/55634).
More preferred proteases are multiply-substituted protease variants. These protease variants comprise a substitution of an amino acid residue with another naturally occuring amino acid residue at an amino acid residue position corresponding to position 103 of *Bacillus amyloliquefaciens* subtilisin in combination with a substitution of an amino acid residue positions corresponding to positions 1, 3, 4, 8, 9, 10, 12, 13, 16, 17, 18, 19, 20, 21, 22, 24, 27, 33, 37, 38, 42, 43, 48, 55, 57, 58, 61, 62, 68, 72, 75, 76, 77, 78, 79, 86, 87, 89, 97, 98, 99, 101, 102, 104, 106, 107, 109, 111, 114, 116, 117, 119, 121, 123, 126, 128, 130, 131, 133, 134, 137, 140, 141, 142, 146, 147, 158, 159, 160, 166, 167, 170, 173, 174, 177, 181, 182, 183, 184, 185, 188, 192, 194, 198, 203, 204, 205, 206, 209, 210, 211, 212, 213, 214, 215, 216, 217, 218, 222, 224, 227, 228, 230, 232, 236, 237, 238, 240, 242, 243, 244, 245, 246, 247, 248, 249, 251, 252, 253, 254, 255, 256, 257, 258, 259, 260, 261, 262, 263, 265, 268, 269, 270, 271, 272, 274 and 275 of *Bacillus amyloliquefaciens* subtilisin; wherein when said protease variant includes a substitution of amino acid residues at positions corresponding to positions 103 and 76, there is also a substitution of an amino acid residue at one or more amino acid residue positions other than amino acid residue positions corresponding to positions 27, 99, 101, 104, 107, 109, 123, 128, 166, 204, 206, 210, 216, 217, 218, 222, 260, 265 or 274 of *Bacillus amyloliquefaciens* subtilisin and/or multiply-substituted protease variants comprising a substitution of an amino acid residue with another naturally occuring amino acid residue at one or more amino acid residue positions corresponding to positions 62, 212, 230, 232, 252 and 257 of *Bacillus amyloliquefaciens* subtilisin as described in PCT application Nos. PCT/US98/22588, PCT/US98/22482 and

PCT/US98/22486 all filed on October 23, 1998 from The Procter & Gamble Company. Preferred multiply substituted protease variants have te amino acid substitution set 101/103/104/159/232/236/245/248/252, more preferably 101G/103A/104I/159D/232V/236H/245R/248D/252K according to the numbering of *Bacillus amyloliquiefaciens subtilisin*.

Amylases (α and/or β) can be included for removal of carbohydrate-based stains. WO94/02597, Novo Nordisk A/S published February 03, 1994, describes cleaning compositions which incorporate mutant amylases. See also WO95/10603, Novo Nordisk A/S, published April 20, 1995. Other amylases known for use in cleaning compositions include both α- and β-amylases.

α-Amylases are known in the art and include those disclosed in US Pat. no. 5,003,257; EP 252,666; WO/91/00353; FR 2,676,456; EP 285,123; EP 525,610; EP 368,341; and British Patent specification no. 1,296,839 (Novo).

Examples of commercial α-amylases products are Purafect Ox Am® from Genencor and Termamyl®, Ban® ,Fungamyl® and Duramyl®, all available from Novo Nordisk A/S Denmark. WO95/26397 describes other suitable amylases : α-amylases characterised by having a specific activity at least 25% higher than the specific activity of Termamyl® at a temperature range of 25°C to 55°C and at a pH value in the range of 8 to 10, measured by the Phadebas® α-amylase activity assay.

Suitable are variants of the above enzymes, described in WO96/23873 (Novo Nordisk). Preferred variants are those demonstrating improved thermal stability, wherein at least one amino acid residue equivalent to F180, R181, G182, T183, G184, or K185 has been deleted from the parent α-amylase. Preferably said variants having improved thermal stability comprise the amino acid deletions R181· + G182·, or T183· + G184·.

Other amylolytic enzymes with improved properties with respect to the activity level and the combination of thermostability and a higher activity level are described in WO95/35382. Other suitable amylases are stability-enhanced amylases described in WO94/18314, published August 18, 1994 and WO96/05295, Genencor, published February 22, 1996 and amylase variants having additional modification in the immediate parent available from Novo Nordisk A/S, disclosed in WO 95/10603, published April 95. Also suitable are amylases described in EP 277 216, WO95/26397 and WO96/23873 (all by Novo Nordisk). Cleaning compositions which incorporate mutant amylases are described in WO94/02597, Novo Nordisk A/S published February 03, 1994. See also WO95/10603, Novo Nordisk A/S, published April 20, 1995.

Further enzymes directed to the degradation of starch are the starch debranching enzymes including pullulanase type I, isopullulanase and isoamylase; the Neopullulanase, the Pullulanase type II, the dextrin dextranase, the cyclomaltodextrin transferase, and the amyloglucosidase.

Starch debranching enzymes

These enzymes are commercially obtainable and examples thereof include SPLENTASE® (manufactured by Amano Pharmaceuticla Co., Ltd.) and PROMOZYME® 200L (manufactured by Novo Industri A/S), as for pullulanase; and "isoamylase" (reagent, manufactured by Seikagaku Kogyo Co., Ltd.), as for isoamylase.

Pullulanase type I enzymes are classified under the IUPAC classification EC 3.2.1.41 and the systematic name α-Dextrin 6-glucanohydrolase. Preferred is pullulanase isolated from a strain of *Bacillus* No. 202-1 as described in Biochimica et Biophysica Acta, 397 (1985) 188-193. Suitable are the alkaline or alkalin-resistant pullulanases described in Japanese Patent Publication (Kokoku) No. 277786/1978; in WO94/19468 and in Japanese Patent Application Laid-open (Kokai) No. 87176/1991. Further suitable pullulanases include the pullulanases from *B. acidopullulyticus* as described in EP 063 909, such as PromozymeTM from Novo Nordisk A/S; the pullulanases described in EP 450 627; WO94/19468; JP04073298 and JP06264094 (all by Kao Corporation). Particularly preferred is the Pullulanase Promozyme (Trade Mark of Novo), isolated from Bacillus sp. Pullulanase.

Isopullulanase enzymes are classified under the IUPAC classification EC 3.2.1.57 and the systematic name Pullulan 4-glucanohydrolase. The isopullulanase enzymes hydrolyse pullulan to isopanose (6-α-maltosylglucose).

Isoamylases are classified under the IUPAC classification EC 3.2.1.68 and the systematic name glycogen 6-glucanohydrolase. Suitable isoamylases are those from *Pseudomonas* species (e.g. *Pseudomonas* sp. SMP1 or *Pseudomonas amyloderomosa* SB15), Bacillus species (e.g. *B. amyloliquefaciens*), *Flavobacterium* species or *Cytophaga* (Lysobacter) species. Isoamylase is commercially available from Sigma, ICN and Hayashibara.

Also suitable are the pullulanase, isopullulanase and iso-amylases enzymes described as starch debranching enzymes in the following Japanese patent/applications: JP 07179900, JP06172796, JP06172792, JP04065494, JP02132193 and JP 02132192, all by the Kao Corporation.

Neopullulanase

A further suitable enzymes is the neopullulanase enzyme EC 3.2.1.135. Neopullulanase enzymes are described in the .following publications: Enzyme Chemistry and Molecular Biology of Amylases and Related Enzyme, pages 28-32 (1995), edited by the Amylases research Society of Japan; H. Takata, et al. J. Biol. Chem., volume 267,

number 26, pages 15447-15452 (1992). Also suitable neopullulanase for use in the present invention is the new neopullulanase Y377F, S422V or M375L which is originated from *Bacillus staerotermophilus* as described in JP07177891; is the neopullulanase from *B. subtilisis* as described in JP06121681;is the neopullulanase from *Bacillus stearothermophilus* TRS (FERM9690), *Thermoactinomyces vulgaris, Bacillus stearothermophilus* KP1064, *Bacteroides thetiaotaomicron* 95-1 and others as described in JP05316992; is the variation-type neopullulanase described in JP04020291 and the neopullulanase described in JP02276578.

Pullulanase type II

Pullulanase type II enzymes are defined as amylopullulanases and hydrolyzes randomly the α,1-4 linkages in addition to the branching points (α-1,6-linkages) in polysaccharides and dextrins. Suitable pullulanase for the purpose of the present invention is the pullulanase described in WO 96/35794; EP 418 835; *Agric. Biol. Chem.,* 51, 9. (1987); Japanese Patent Publication No. 18717/1989 and in JP04072397.

Dextrin dextranase

A further suitable enzyme from the amylase class, is the dextrin dextranase (EC 2.4.1.2). Dextrin dextranase is described in Enzyme Chemistry and Molecular Biology of Amylases and Related enzymes, edited by the Amylase Research Society of Japan, 1995 CRC Press.

Cyclomaltodextrin glucanotransferase

Also suitable is the cyclomaltodextrin glucanotransferase, EC 2.4.1.19. Commercially available cyclomaltodextrin glucanotransferase enzymes are sold under the tradenames CGTase by Amano; EN301 by Hayashibara and Toruzyme by Novo Nordisk A/S.

Amyloglucosidase

The amyloglucosidase is classified under EC 3.2.1.3. and its systematic name is 1,4-α-D-glucan glucohydrolase. Commercially available amyloglucosidases are the enzyme products sold under the trademane PALKODEX by MAPS; AMG300L by Novo Nordisk A/S, Optimax 7525 (Combinations of enzymes including amyloglucosidase) and Spezyme by Genencor. Further commercial available amyloglucosidases are those from *Aspergillus niger* obtainable from the following companies: Ambazyme, Amano, Boehringer, Fluka, Sigma, Aldomax, Genzyme, Nagase, UOP. Also suitable are the amyloglucosidases from *Aspergillus* species from the companies Biocatalysts or Danisco and the amyloglucosidases from *Rhizopus delemar* from Nagase; from *Rhizopus niveus* from Amano, ICN, Seikagaku; from *Rhizopus oryzae* from Enzyme Development Co-operation.

Further suitable enzymes for the present invention are the transferases represented by the EC 2.1 Transferring one-carbon groups enzymes, EC 2.2 Transferring aldehyde or ketone residues enzymes, EC 2.3 Acyltransferases, EC 2.4 Glycosyltransferase, EC 2.5 Transferring alkyl or aryl groups other than methyl groups enzymes, EC 2.6 Transferring nitrogenous groups enzymes and EC 2.7 Transferring phosphorus-containing groups enzymes; preferably the acyl transferases (EC 2.3) and glycosyl transferases classes ( EC 2.4).

Also suitable are mutant glycosyltransferases and/or mutant glycosidases, examples of which are described in PCT Application Publication No. WO 97/21822 to S.G. Withers Protein Eng. Net. Canada. Other enzymes that are of particular interest is endoxyloglucan transferase ("EXT"), which is described in J. Plant Res. 108, 137-148, 1995 by Nishitani, Kagoma University, and now called "EXGT" in Int. Review of Cytology, Vol. 173, p. 157, 1997 by Nishitani, Kagoma University and the xyloglucan endotransglycosylase ("XET") which is described in Novo Nordisk patent application WO97/23683. Yet another enzyme that is of particular interest is cyclomaltodextrin glucanotransferase ("CGTase") (EC 2.4.1.19), which is commercially available from Amano and Novo Nordisk A/S. Yet still another group of enzymes that is of particular interest is glucansucrases, of which dextransucrase (EC 2.4.1.5), a glycosyltransferase, is one example. Other glucansucrases that are suitable for use in herein include, various dextransucrases, altemansucrase and levansucrase.

Cell wall degrading enzymes are suitable for the purpose of the present invention. They are generally distributed into three broad enzyme classes of cellulases, hemicellulases and pectinases (Ward and Young (1989), CRC Critical Rev. in Biotech. 8, 237-274). Cellulolytic enzymes have been traditionally divided into three classes : endoglucanases, exoglucanases or cellobiohydrolases and β-glucosidases (Knowles, J. et al. (1987) TIBTECH 5, 255-261). Examples of pectinases are pectin esterase, pectin lyase, pectate lyase and endo- or exo-polygalacturonase (Pilnik and Voragen (1990) Food Biotech 4, 319-328), enzymes degrading hairy regions such as rhamnogalacturonase and accessory enzymes (Schols et al. (1990), Carbohydrate Res. 206, 105-115; Searle Van Leeuw et al. (1992) Appl.

Microbiol. Biotech. 38, 347-349). Galactanase, arabinase, lichenase and mannase are some hemicellulose degrading enzymes of interest.

Suitable cellulases include both bacterial or fungal cellulases. Preferably, they will have a pH optimum of between 5 and 12 and a specific activity above 50 CEVU/mg (Cellulose Viscosity Unit). Suitable cellulases are disclosed in U.S. Patent 4,435,307, Barbesgoard et al, J61078384 and WO96/02653 which discloses fungal cellulase produced respectively from *Humicola insolens, Trichoderma, Thielavia* and *Sporotrichum*. EP 739 982 describes cellulases isolated from novel Bacillus species. Suitable cellulases are also disclosed in GB-A-2.075.028; GB-A-2.095.275; DE-OS-2.247.832 and WO95/26398.

Examples of such cellulases are cellulases produced by a strain of *Humicola insolens (Humicola grisea* var. *thermoidea),* particularly the *Humicola* strain DSM 1800. Other suitable cellulases are cellulases originated from Humicola insolens having a molecular weight of about 50KDa, an isoelectric point of 5.5 and containing 415 amino acids; and a ~43kD endoglucanase derived from *Humicola insolens*, DSM 1800, exhibiting cellulase activity; a preferred endoglucanase component has the amino acid sequence disclosed in PCT Patent Application No. WO 91/17243. Also suitable cellulases are the EGIII cellulases from *Trichoderma longibrachiatum* described in WO94/21801, Genencor, published September 29, 1994. See also WO91/17244 and WO91/21801. Other suitable cellulases for fabric care and/or cleaning properties are described in WO96/34092, WO96/17994 and WO95/24471.

By pectin degrading enzyme it is meant any enzyme which acts to break down pectic substances and pectin related substances and emcompass polygalactironase (EC 3.2.1.15), exopolygalacturonase (EC 3.2.1.67), exo-poly-α-galacturonidase (EC 3.2.1.82), pectin lyase (EC 4.2.2.10), pectin esterase (EC 3.2.1.11. pectate lyase (EC 4.2.2.2), exo-polugalacturonate lyase (EC 4.2.2.9) and hemicellulase such as endo-1,3-β -xylosidase (EC 3.2.1.32), xylan-1,4-β-xylosidase (EC 3.2.1.37 and α-L-arabinofuranosidase (EC 3.2.1.55).

In particular, pectate lyase are suitable pectin degrading enzyme suitable for the present invention, which is classified as EC 4.2.2.2. Said enzyme is preferably subtantially free for other pectin degrading activities, i.e having less than 25%, preferably less than 15%, more preferably less than 5% by weight of the enzyme compound of other pectin degrading enzyme activities. Further suitable pectate lyases are the protopectinases having an optimum reaction pH of 7.0 or higher when polygalacturonic acid is used as a substrate such as described in WO98/45393 and the pectic acid lyase having the amino acid sequence SEQ no 1 of EP 870 843 or having such amino acid sequence with one or more amino acid being deleted, added or substituted. Preferred are the pectate lyase enzymes described in the international co-pending application PCT/DK98/00515, filed internationally on November 24, 1998; and the pectate lyase enzyme described in the international co-pending application PCT/DK98/00514, filed internationally on November 24, 1998.

Also suitable for the purpose of the present invention are the enzymes as described in WO99/09127 : Arabinases : Endo Arabanase (E.C. 3.2.1.99), such as endo a-1,5-arabinosidase, exo Arabanase (E.C. 3.2.1.55), exo A (α-1,2; α-1,3) arabinofuranosidase, exo B (α-1,3; α-1,5) arabinofuranosidase; (α-1,2; α-1,3) fucosidase, a-1,6-fucosidase (E.C. 3.2.1.127); β-1,2-Galactanase, β-1,3-Galactanase (E.C. 3.2.1.90), β-1,4-Galactanase, β-1,6-Galactanase, Galactanase are a also called Arabino galactan galactosidase (E.C. 3.2.1.89), α and β galactosidase (E.C. 3.2.1.22 & 23), (E.C. 3.2.1.102) (E.C. 3.2.1.103); β-Mannosidase (3.2.1.25), α-Mannosidase (3.2.1.24), β-1,2-Mannosidase, α-1,2-Mannosidase (E.C. 3.2.1.113) (E.C. 3.2.1.130), α-1,2-1.6 -Mannosidase (3.2.1.137), β-1,3-Mannosidase (E.C. 3.2.1.77), β-1,4-Mannosidase (E.C. 3.2.1.78), β-1,6-Mannosidase (E.C. 3.2.1.101), α-1,3-1,6-Mannosidase (E.C. 3.2.1.114), β-1,4-Mannobiosidase (E.C. 3.2.1.100); Glucuronosidase (E.C. 3.2.1.131), glucuronidase (E.C. 3.2.1.31). exo 1,2 or 1,4 glucuronidase, Agarase (E.C. 3.2.1.81), Carrageenase (E.C. 3.2.1.83), a-1,2-, Xanthan lyase; Poly(α-L guluronate) lyase , also called Alginase II (E.C. 4.2.2.11).

These enzymes are commercially available : the galactomannanase sold under the tradename Gammanase® and the arabanase sold under the trade name Pectinex AR by Novo Nordisk A/S. Also are the enzymes sold under the tradenames the Pectinex Ulta SP by Novo Nordisk A/S, Rapidase Pineapple by Gist -Brocades, Rohapec B1L by Rohm; all enzymatic preparations having a galactomannanase, arabinogalactanase, galactoglucomannanase and/or arabinoxylanase activity. Also available is the saccharide gum degrading enzyme sold under the tradename Rapidase light by Gist-Brocades and endo-galactanase form Megazyme Ltd (Australia).

Aslo suitable is the xyloglucanase described in WO98/50513, this is an enzyme exhibiting endoglucanase activity specific for xyloglucan. As used herein, the term "endoglucanase activity" means the capability of the enzyme to hydrolyze 1,4-β-D-glycosidic linkages present in any cellulosic material, such as cellulose, cellulose derivatives, lichenin, β-D-glucan, or xyloglucan; which may be determined in accordance WO 94/14953. The term "specific for xyloglucan" means that the endoglucanse enzyme exhibits its highest endoglucanase activity on a xyloglucan substrate, and preferably less than 75% activity, more preferably less than 50% activity, most preferably less than about 25% activity, on other cellulose-containing substrates such as carboxymethyl cellulose, cellulose, or other glucans. A further suitable xyloglucanase is described in WO99/02663 by Novo Nordisk A/S.

Aslos suitable for the present invention are the following mannans-degrading enzymes : EC 3.2.1.25 : β-mannosidase, EC 3.2.1.78 : Endo-1,4-β-mannosidase, referred therein after as "mannanase" and EC 3.2.1.100 : 1,4-β-mannobiosidase ; preferably a β-1,4-Mannosidase (E.C. 3.2.1.78) referred to as Mannanase. Preferred mannanases are the mannanases described in the intenational patent application PCT/DK99/00314 filed internationally on June 10, 1999 : the alkaline mannanase selected from the strain *Bacillus agaradhaerens* NICMB 40482; the mannanase from *Bacillus subtilis* strain 168,; the mannanase from *Bacillus sp.* 1633 and/or the mannanase from *Bacillus sp.* AAI12, more preferred is the mannanase enzyme originating from *Bacillus sp.* 1633.

Also suitable enzymes for the purpose of the present invention are the following enzymes as described in the following patent applications filed by the Procter & Gamble Company : the xylan degrading enzymes as described in the co-pending application published under EP 709 452 A; the cholesterol esterase EC 3.1.1.13 as described in WO93/10224 and in WO94/23052 by Novo Nordisk A/S; Keratanase EC 3.2.1.103 as described in the published application EP 747 470 A; the chondroitinase EC 4.2.2.4, EC 4.2.2.5 and EC 4.2.2 as described in the published application EP 747 469 A; the bleaching enzymes including the peroxidase as described in WO89/099813, WO89/09813, EP 540 784 A and WO 97/30143, the laccase EC 1.10.3.2 as described in the published patent application WO97/43384, the catechol oxidase EC 1.10.3.10, the bilirubin oxidase EC 1.3.3.5, the monophenol monooxygenase EC 1.14.99.1; the cytochrome EC 1.14.13, EC 1.14.14, EC 1.14.15 and EC 1.14.99. as described in copending patent application WO 99/02641 and in the co-pending patent application US serial No. US97/12445; the specific oxygenases described in the co-pending patent applications WO 99/02639, WO 99/2632 and WO99/02638 : polyphenol / heterocyclic substrate based oxygenase enzyme as described WO99/02639; the proteinic substrate based oxygenase as described in WO99/02632; the oxygenase directed to body soils as described in WO99/02638; the endo-dextranase as described in the copending EP 883 673 A; the mycodextranase as described in the co-pending application EP 929 635 A; the hyaluronidase EC 3.2.1.35, EC 3.2.1.36 and EC 4.2.2.1. as described in the published patent application WO 97/24426; the hexosaminidase as described in the copending patent application WO98/50512 .

The above-mentioned enzymes may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, etc.). Purified or non-purified forms of these enzymes may be used. Nowadays, it is common practice to modify wild-type enzymes via protein / genetic engineering techniques in order to optimise their performance efficiency in the cleaning compositions of the invention. For example, the variants may be designed such that the compatibility of the enzyme to commonly encountered ingredients of such compositions is increased. Alternatively, the variant may be designed such that the optimal pH, bleach or chelant stability, catalytic activity and the like, of the enzyme variant is tailored to suit the particular cleaning application.

In particular, attention should be focused on amino acids sensitive to oxidation in the case of bleach stability and on surface charges for the surfactant compatibility. The isoelectric point of such enzymes may be modified by the substitution of some charged amino acids, e.g. an increase in isoelectric point may help to improve compatibility with anionic surfactants. The stability of the enzymes may be further enhanced by the creation of e.g. additional salt bridges and enforcing metal binding sites to increase chelant stability.

The enzymes can be used as separate single ingredients in prills, granulates, stabilised liquids, etc. forms containing one enzyme or as mixtures of two or more enzymes (e.g. cogranulates). The enzymes to be linked to the Mimic CBD according to the present invention can already comprise a natural CBD; a Mimic CBD will be added.

As described above, when the benefit agent is indeed an enzyme, such enzyme chemical entity can be prepared by Genetic Engineering. Enzyme hybrids are known in the art, see e.g. WO 90/00609 and WO 95/16782, and may be prepared by transforming into a host cell a DNA construct comprising at least a fragment of DNA encoding the cellulose-binding domain ligated, with or without a linker, to a DNA sequence encoding the enzyme and growing the host cell to express the fused gene. Enzyme hybrids may be described by the following formula:

$$\text{CBD - MR - X}$$

wherein CBD is a Mimic CBD according to the present invention; MR is the middle region (the linker), and may be a bond, or a short linking group preferably of from about 2 to about 100 carbon atoms, more preferably of from 2 to 40 carbon atoms; or is preferably from about 2 to to about 100 amino acids, more preferably of from 2 to 40 amino acids; and X is an N-terminal or C-terminal region of the enzyme.

***Perfumes***

A chemical entity suitable for the compositions of the present invention is a perfume entity wherein at least one benefit agent is a perfume compound linked to a Mimic CBD via a weak bond. It is believed that high substantivity of the perfume will be achieved by linking the perfume agents to a Mimic CBD that very effective and cost-performance deposition of the perfume will be achived via the Mimic CBD.

Without wishing to be bound by theory, it is believed that the slow hydrolysis of the weak bond within the

improved perfume entity will improve the release of the perfume. Indeed, after the wash or fabric care process, the weak bond will be hydrolysed and the perfume will be released. It has been surprisingly found that these perfume entities in the compositions of the present invention provide increased pleasing and long-lasting fragrance, through very economic and effective means.

Fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation. In this list of perfume ingredients, some are commercial names conventionally known to one skilled in the art, and also includes isomers. Such isomers are also suitable for use in the present invention. A typical disclosure of suitable ketone and/or aldehydes, traditionally used in perfumery, can be found in "perfume and Flavor Chemicals", Vol. I and II, S. Arctander, Allured Publishing, 1994, ISBN 0-931710-35-5. Preferred for the purpose of the present invention are the aldehydes or ketones based products.

- Natural products such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil;
- Alcohols such as famesol, geraniol, linalool, nerol, phenylethyl alcohol, rhodinol, cinnamic alcohol;
- Aldehydes such as citral, Helional™, alpha-hexyl-cinnamaldehyde, hydroxycitronellal, Lilial™ (p-tert.butyl-alpha -methyldihydrocinnamaidehyde), methylnonylacetaldehyde, 1-decanal, benzaldehyde, florhydral, 2,4-dimethyl-3-cyclohexen-1-carboxaldehyde; cis/trans-3,7-dimethyl-2,6-octadien-1-al; heliotropin; 2,4,6-trimethyl-3-cyclohexene-1-carboxaldehyde; 2,6-nonadienal; alpha-n-amyl cinnamic aldehyde, P.T. Bucinal, lyral, cymal, methyl nonyl acetaldehyde, hexanal, trans-2-hexenal, and mixture thereof;
- Ketones such as allylionone, alpha-ionone, beta -ionone, isoraldein (isomethyl- alpha - ionone), methylionone, Alpha Damascone, Delta Damascone, Iso Damascone, Carvone, Gamma-Methyl-Ionone, Iso-E-Super, 2,4,4,7-Tetramethyl-oct-6-en-3-one, Benzyl Acetone, Beta Damascone, Damascenone, methyl dihydrojasmonate, methyl cedrylone, and mixtures thereof;
- Esters such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc.;
- Lactones such as gamma-undecalactone, various components often used in perfumery, such as musk ketone, indole, p-menthane-8-thiol-3-one, and methyl-eugenol;
- Acetals and ketals include the well-known methyl and ethyl acetals and ketals, as well as acetals or ketals based on benzaldehyde, those comprising phenylethyl moieties, or more recently developed specialties such as those described in a United States Patent entitled "Acetals and Ketals of Oxo-Tetralins and Oxo-Indanes, see U.S. Pat. No. 5 ,084,440, issued January 28, 1992, assigned to Givaudan Corp. ;
- Recent synthetic specialties include the enol ethers of alkyl-substituted oxo-tetralins and oxo-indanes as described in U.S. Pat. 5,332,725, July 26, 1994, assigned to Givaudan; or Schiff Bases as described in U.S. Pat. 5,264,615, December 9, 1991, assigned to Givaudan.

When encompassed in the compositions of the present invention, these perfume entities will be generally comprised at a level of from 0.001% to 20%, preferably from 0.005% to 5% by weight of the total composition.

***Hygiene agent***

A further suitable chemical entity for the present invention is a hygiene entity wherein at least one benefit agent is a hygiene agent, linked to a Mimic CBD. The hygiene agent will be linked to the Mimic CBD and/or linking region via weak bond in order to release the active material upon time during or after the wash or fabric care process. Preferably, the hygiene agent comprises an hydroxyl, carboxyl or aldehyde reactive moiety.

It has been surprisingly found that such hygiene entities in the laundry detergent and/or fabric care compositions of the present invention, provide an extremely long lasting and very efficient control of the micro-organism growth on stored and weared fabrics. It is believed that very effective and cost-performance deposition of the hygiene agent will be achived via the Mimic CBD.

Sanitisation includes all positive effects obtained by the inhibition or reduction of microbial activity on fabrics and other surfaces, such as the prevention of malodour development and bacterial/fungal growth. For example, it provides prevention of malodour development on stored and weared fabrics. In particular, the composition of the invention will inhibit or at least reduce the bacterial and/or fungal development on moist fabric waiting for further laundry processing and thereby preventing the formation of malodour. The term hygiene agents herein encompasses fungicides and antimicrobials that when applied to fabric respectively prevent or reduce the growth of fungi or bacteria. The sanitisation benefits of the laundry detergent and/or fabric care compositions of the present invention can be evaluated by the Minimum Inhibitory Concentration (MIC) as described in Tuber. Lung. Dis. 1994 Aug; 75(4):286-90; J. Clin. Microbiol. 1994 May; 32(5):1261-7 and J. Clin. Microbiol. 1992 Oct; 30(10):2692-7.

Preferred antibacterial compounds are pentadecanol, cinamaldehyde, ionone, glutaraldehyde, citronellal. Other suitable antimicrobial compounds with an hydroxyl, carboxyl or aldehyde moiety are described in Parfums Cosmétiques Actualités No 125, Nov, 1995, 51-4. Other suitable antibacterial components are the Nerodol which can for example be linked to the carboxylic groups or succinic acid linked to the alcohol group of the Mimic CBD and/or linking region. A further example are the compounds Cipamaldehyde and/or Beta ionone which can form for example a Shiff base or β-amino ketone with the NH2 groups of the Mimic CBD and/or linking region.

Also suitable are the microbicidally active ingredients described in the handbook of Disinfectants and Antiseptics edited by J.M. Ascenzi and in WO97/46218 such as 2-hydroxydiphenyl ether, phenol derivatives, diphenyl compounds, benzyl alcohols, chlorhexidine, C12-14 alkylbetaines and C8-18 fatty acid amido alkylbetaines, amphoteric surfactants, trihalocarbanilides and quaternary ammonium salts. Also suitable are the cationic germicides described in EP 843 002 and in WO98/24314 and the antibacterial agents triclosan, tridocarban, DMDM hydantoin, piroctone olamine, zinc pyrithione, selenium disulfide, climnazole and 3-methyl-4-(1-methylethyl)phenol also therein described. Other examples of suitable fungicides are given in WO94/10286 (Henkel), CA943 429 (Unilever) and US3,426,024 (Henkel). Preferred antimicrobials are 2-thiocyanomethylthiobenzothiazole (Busan 30 WB ex Buckmann), butyl 4-hydroxybenzoate (Butyl Parabens ex Nipa Labs), propyl 4-hydroxybenzoate (Propyl Parabens ex Nipa Labs), Terpineol, Bomeol, Fenchyl alcohol, trichlorocarbanilidem, Irgasan DP300 (2,4,4'-trichloro 2'hydroxydiphenylehter) and the higher homologues of hydroxybenzoate esters. Further examples of bactericides used in the hygiene agent entities of this invention include formaldehyde, 2-bromo-2-nitro-propane-1,3-diol sold by Inolex Chemicals, located in Philadelphia, Pennsylvania, under the trade name Bronopol ®, and a mixture of 5-chloro-2-methyl-4-isothiazoline-3-one and 2-methyl-4-isothiazoline-3-one sold by Rohm and Haas Company under the trade name Kathon.

Further suitable hygiene agents include : 1- or 2-hexadecanol, 2-tetradecanol, 1-pentadecanol, 1-Undecanol, 2-dodecanol, 1-Tridecanol, nerolidiol, hinokitiol, tropolone, berberine, citronellic acid, Curcumin, 2-Mercaptopyridine N-oxide, Ellagic acid (dihydrate), 3-t-Butyl-5-methyl salicylic acid, 3-, 4- or 5-methyl salicylic acid, 1-nonanol, Decylalcohol, Cinamaldehyde, S- or R-Citronellal, Citronellol, Beta-ionone, Thujone, Coumarin and derivatives, Geraniol, Citral, Thymol, Iso-butyl- or Isopropyl-quinoline, 2-butyl-5-methylphenol, 2-Mercapto-3-pyrodinol, Perillyl alcohol, 6-hydroxy-1,3-benzoxathiol-2-one, BOAT, (Iso)-Eugenol, Menth-1-en-9-ol, 2-t-Butyl-4-methylphenol, Kojic acid, Camphene, Carveol, Dihydroxycarvecol, Isojasmone, Methol, Cineol, Terpinol, Camphor, 2-t-Butyl-methyl phenol, 2-Tridecanone, Acetyl salicylic acid, Salicylaldoxime, Undecyclenic aldehyde, Nerol, 3,5,5-trimethyl-1-hexanol, Adipic acid, Thiosalicylic acid, OH-Benzoic acid, 2-Methylbenzothiazole, 2-Aminobenzothiazole, Caryophyllene, Allyl-isocyanate, Carvone, Alpha-pinene, Salicylic acidfrole, Alpha-ionone, 20H- or 30H-Phenethyl alcohol, Trimethoxy BP, Undecylic aldehyde, Cineole, Anisaldehyde, Bomyl acetate, Salicylhydroxamic, Benzofuran Car., Syringaldehyde.

Preferably, the levels of the hygiene entity should be such that they prevent bacterial and fungi growth on fabrics, rather than merely preventing growth within the laundry detergent and/or fabric care compositions per se. When encompassed in the compositions of the present invention, these hygiene entities will be generally comprised at a level of from 0.00001% to 20%, preferably from 0.00001% to 5% by weight of the total composition.

***Insect Control agent***

Further suitable chemical entities for the compositions of the present invention are insect control entities wherein at least one benefit agent is an insect control agent, linked to a Mimic CBD.

It has surprisingly been determined that such insect control entities in the laundry detergent and/or fabric care compositions of the present invention provide a much longer lasting insect control by the slow release of the insect control agent. Indeed, the very effective binding of the insect agent via the Mimic CBD provides enchanced deposition and insect control performance.

Such insect control agents are linked to the Mimic CBD and/or linking region preferably via a weak bond in order to release upon time the active material. For example, these materials can be linked to the NH2 group present in the Mimic CBD and/or linking region via a Shiff base or Michael reaction.

The term insect control agent refers to both insecticides and insect repellents either individually or as mixtures. Examples of insect repellents can be found in Kirk-Othmer Encyclopedia of Chemical technology, Fourth edition, volume 13, pages 474 to 478. Suitable insect repellents include aldehyde based compounds such as Citronellal and Rotundial, ene one based compounds such as Butopyronoxyl (Indalone TM), benzyl benzoate, bioallethrin and dimethrin, N,N-diethyl toluamide ("DEET"), N,N-diethyl benzamide, p-menthane-3,8-diol, 1S,3S,4S,6S-carene-3,4diol (Sumitomo - US5,130,136), 1-piperidinecarboxylic acid, 2-(2-hydroxyethyl)-, 1-methylpropylester, 1-(3cyclohexen-1-yl carbonyl)-2methylpiperidine, 1-(3-cyclohexen-1-yl carbonmyl) piperidine, N,N-diethyl mandelamide, isopulegol hydrate, ethyl-3 (N-butyl-N-acetyl) aminopropionate, diisopropyladipate, α-biasal, psearmint oil, benzyl alcohol, N,N-diethylphenylacetamide, vitamin E, citronella oil, coconut oil, cedar oil, geraniol, lemon grass oil, thyme oil, reosemary oil, mint oil, geranium oil, eugenol, 3-acetyl-2-(2-,6-dimethyl-5-heptenyl) oxazolidine, (2-hydroxymethylcyclohexyl)acetic acid lactone and eucalyptol. Other insect control agents are based on pyrethroid insecticides, in particular 3-phenoxybenzyl-DL-cis, trans-3-(2,2-dichlorovinyl)-2,2-dimethylcyclopropanecarboxylate (permethrin). WO98/17772 describes the insect repellent agent 3-(N-butylacetamino) ethyl propionate for use in detergents.
Preferred insect repellents are aldehyde based compounds such as Citronellal and Rotundial.
When encompassed in the compositions of the present invention, these insect control entities are generally comprised at a level of from 0.1% to 40%, preferably from 0.1% to 10% by weight of the total composition.

***Bleaching agent***

A further suitable chemical entity for the present invention is a bleaching entity wherein at least one benefit agent is a bleaching agent, linked to a Mimic CBD. Said bleaching agent can be selected from hydrophilic bleach activators, hydrophobic bleach activators, metal catalysts and/or photoactivated bleaches.
It has been surprisingly found that the bleaching action with such bleaching entities within the laundry detergent and/or fabric care compositions of the present invention, results in improved and increased stain/soil bleaching/ removal and whiteness maintenance. Without wishing to be bound by theory, it is believed that improved stain/soil bleaching/removal and whiteness maintenance results in the generation of oxygen radicals, peracetic acid or peracid perhydrolysis occurring very close to the fabric surface. This effect is enhanced by the very effective binding of the Mimic CBD. Said peracetic acid or peracid is less dilute in the wash solution due to the bleach entity of the present invention being very efficiently deposited onto the fabric and thereby improving the bleaching action on the fabric surface.
Preferably, the bleach activator is linked to the Mimic CBD via a linking region, more preferably via a polyreactive linking region. Without wishing to be bound by theory, it is believed that the presence of such a linking region provides flexibility of movement to the bleach activator therefore enabling it to perform more efficiently its bleaching action on the fabric fibers.

1) Hydrophilic bleach activator : For example, Tetra Acetyl Ethylene Diamine (TAED) is a current bleach activator used in laundry detergent and forms peracetic acid in presence of H2O2. It is known that the two NH2 groups of the Ethylene Diamine are diacetylated to form TAED. Without wishing to be bound by theory, it is believed that the diacetylation of the free NH2 groups of the Mimic CBD and/or linking region, forms a substantive fabric hydrophilic bleach activator. Hydrophilic bleach activator of the TAED type can be linked for example via a diacetylation reaction to any NH2 groups of the Mimic CBD and/or linking region of the present invention.

2) Hydrophobic bleach activators such as Nonanoyl benzene sulphonate, are current bleach activators used in laundry detergent. It has been surprisingly found that the phenolic moiety can be replaced by the one present in the tyrosine amino acid present in the Mimic CBD and/or linking region. Active hydrophobic bleach activator precursors such as nonanyl unit can be linked to the phenol group of an amino acid such as tyrosine present in the peptidic Mimic CBD and/or linking region. Similarly, if there is no phenol entity available in the Mimic CBD and/or linking region, a phenol unit can be linked to a group NH2 in this Mimic CBD and/or linking region. Hydrophobic bleach activators in general can be linked to the Mimic CBD and/or linking region for example by a reaction on NH2 groups.
Other examples of suitable hydrophobic bleach activators are : nonanoyloxybenzene-sulfonate (NOBS, described in US 4,412,934), 3,5,-trimethylhexanoloxybenzenesulfonate (ISONOBS, described in EP 120,591) or pentaacetylglucose (PAG) or Phenolsulfonate ester of N-nonanoyl-6-aminocaproic acid (NACA-OBS, described in WO94/28106), which are perhydrolyzed to form a peracid as the active bleaching species, leading to improved bleaching effect.
Also suitable bleach activators are the activators based on a caprolactam leaving group such as benzoyl caprolactam and quaternary ammonium hexanoyl caprolactam; the imides activators such as N-nonanoyl-N-methyl acetamide and unsymetrical acyclic imide bleach activator of the following formula as disclosed in the Procter & Gamble copending patent applications US serial No. 60/022,786 (filed July 30, 1996) and No. 60/028,122 (filed October 15, 1996) :

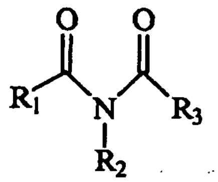

wherein $R_1$ is a $C_7$-$C_{13}$ linear or branched chain saturated or unsaturated alkyl group, $R_2$ is a $C_1$-$C_8$, linear or branched chain saturated or unsaturated alkyl group and $R_3$ is a $C_1$-$C_4$ linear or branched chain saturated or unsaturated alkyl group. These bleach activators can for example be linked to any NH2 groups of the polyreative linking region. Also suitable are the pre-fromed peracid such as nonylamido peroxy adipic acid and N,N-phtaloylaminoperoxy caproic acid and the Di-acyl peroxides such as dobenzoyl peroxide.

3) Metal catalysts : A third type of bleach activator that can be encompassed in the bleaching entity of the present invention, are the below described metal catalysts. For example, these catalysts can be linked via their capping cyclo azo moieties to NH2 groups of the Mimic CBD and/or linking region.
Examples of Metal-containing catalysts for use in bleach compositions, include cobalt-containing catalysts such as Pentaamine acetate cobalt(III) salts and manganese-containing catalysts such as those described in EPA 549 271; EPA 549 272; EPA 458 397; US 5,246,621; EPA 458 398; US 5,194,416 and US 5,114,611. Bleaching composition comprising a peroxy compound, a manganese-containing bleach catalyst and a chelating agent is described in the patent application No 94870206.3.

4) Photoactivated bleach : Another group of suitable bleach activators that can be encompassed in the bleaching entity of the present invention, are the photoactivated bleaches. For example, the linking can be achieved between the NH2 groups of and the benzyls groups of these photoactivated bleaches, activated via a preliminary bromination.
Suitable photoactivated bleaching agents are the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, the compositions herein will contain about 0.025% to about 1.25%, by weight of sulfonated zinc phthalocyanine.

In addition to this bleaching entity, the compositions of the present invention can further comprise bleaching species such as hydrogen peroxide, PB1, PB4 and percarbonate, typically be present at levels of from about 1% to about 25% by weight.
When encompassed in the compositions of the present invention, these bleaching entities will be generally comprised at a level of from 0.001% to 40%, preferably from 0.1% to 10% by weight of the total composition.

***Fabric softening agents***

As described above the Mimic CBDs of the present invention can be used per se, as fabric care benefits agents. The Mimic CBD or the cross-linked Mimic CBDs can be further linked to a softening protein. Suitable softening proteins for the chemical entity of the present invention are enzymes proteins (EP 687 729), polyamino acid resin solution (JP01280079), and/or the wheat protein derivatives.
Enzyme proteins to be used herein are those generically defined as a class of proteins having a particular structure for catalytic action. In other words, all proteins that possess a structure for catalytic action can be used, whether or not they exhibit catalytic action. However, enzyme proteins suitable herein are inactive. Inactivation can occur for example by inhibition, by the distortion of the three-dimensional structure for example by thermal or chemical means. When proteins other than enzyme proteins are used, the effects of the present invention may be obtained to some extent, but sufficient effect may not be achieved. Enzyme proteins have different biological origins: animal, plant and microbial origins. Enzyme proteins of any origin are usable for the present invention.
Such enzyme proteins, as classified on the basis of enzyme reaction type, include hydrolases, lyases, oxidoreductases, ligases, transferases and isomerases, all of which are usable for the present invention. A preference is given to hydrolases, exemplified by proteases (peptidase), glucosidases such as cellulase and amylase, and esterases such as lipase.
The molecular weight of the enzyme protein is preferably not lower than 10,000, more preferably in the range of from

20,000 to 300,000. Being not lower than 10,000 in molecular weight, some enzyme proteins cannot penetrate the single fiber/monofilament (lamella structure) of cellulosic fibers such as a natural cellulose fiber and rayon. Also, they may not penetrate the monofilament of synthetic fibers, because the monofilament internal structure is dense. Enzyme adsorption sites of cellulosic fibers and synthetic fibers are therefore limited to the surface of the single fiber/monofilament.

Preferably the softening enzyme protein will be chosen from inactive enzyme comprising a CBD in nature, such as cellulase, xylanases, mannanases, arabinofuranosidases, acetylesterases and chitinases.

JP01280079 describes another type of softening proteins that can be used herein. These softening proteins are polyamino acid resin solution that adhere to synthetic, semi synthetic or cotton fabrics and thereby provide softness. These polyamino acids are preferably alpha amino acid such as glutamic acid, glycine, omithine, mono- or copolymer such as poly-gamma-L-glutamate.

Also suitable are the C18 alkyl quaternary wheat protein derivatives sold under the tradename Coltide HQS by Croda Colloids Ltd. These wheat proteins derivatives generally included at levels of 0.04% to 0.2% by weight, are known to provide great conditioning effect, i.e. to provide great handfeeling, softeness, to prevent fibres erosion of cotton and wool fabrics and to increase the lubricity of wool fibres.

Also encompassed in the present invention are the fabric softening entities wherein at least one benefit agent is a fabric softening agent, linked to a Mimic CBD. It has been surprisingly found that such fabric softening entities within the laundry detergent and/or fabric care compositions of the present invention, provide anti-wrinkling, anti-bobbling and anti-shrinkage properties, as well as fabric softness. Without wishing to be bound by theory, it is believed that the very effective binding via the Mimic CBD of the fabric softening entity to the fabric results in increased accessibility of the fabric softening entity as well as of the $Ca^{++}$ salts to the fabric surface. These $Ca^{++}$ salts are known to give a "harsh" feeling to the fabric.

Indeed, recent years, consumer desirability for durable press fabric garments, particularly cotton fabric garments, has risen. Durable press garments include those garments which resist wrinkling of the fabric both during wear and during the laundering process. Durable press garments can greatly decrease the hand work associated with laundering by eliminating ironing sometimes necessary to prevent wrinkling of the garment. However, in most commercially available durable press fabrics, the fabric's ability to resist wrinkling is reduced over time as the garment is repeatedly worn and laundered. It has been found that the fabric softening entities of the present invention provide excellent anti-wrinkling, anti-bobbling and anti-shrinkage properties, as well as fabric softness properties due to very effective deposition of the fabric softening entity on the fabric with the combined effect of the surfactant and protease.

Suitable fabric softening agents are dialkyl units that can be linked for example, by dialkylation of the NH2 groups comprised in the Mimic CBD and/or linking region. Preferably such fabric softening entities will not comprise a weak bond.

Dialkyl units suitable for the compositions of the present invention can be extracted from the following cationic softening surfactants currently used in the laundry detergent and/or fabric care context.

The alkyl, or alkenyl chain will contain at least 11 carbon atoms, preferably at least 16 carbon atoms. The chain may be straight or branched. Specific examples of the alkyl or alkenyl chains herein include :

1) N,N-di(tallowyl-oxy-ethyl);
2) N,N-di(2-tallowyloxy-2-oxo-ethyl);
3) N,N-di(2-tallowyloxyethylcarbonyloxyethyl);
4)N-(2-tallowoyloxy-2-ethyl)-N-(2-tallowyloxy-2-oxo-ethyl);
5) N-(2-tallowyloxy-2-oxoethyl)-N-(tallowyl); and
6) 1,2-ditallowyloxy;

and mixtures of any of the above materials.

Preferred alkyl chains for the purpose of the present invention N,N-di(tallowoyl-oxyethyl), where the tallow chains are at least partially unsaturated.

Other suitable fabric softening agents include quaternary ammonium softening compounds having a solubility in water at pH2.5 and 20°C of less than 10g/l. It is particularly advantageous if the fabric softening agent is a quaternary ammonium compound in which at least one long chain alkyl group is connected to the quaternary ammonium compound via at least one ester link. Suitable cationic softeners are described in US4,137,180 (Naik) and WO93/23510.

Also suitable as softening components are clay or silicone.

Suitable clays include a three layered smectite clay, preferably having a cationic exchange capacity as described in GB 1,400,898 and in USP 5,019,292. Especially preferred are clays which are 2:1 layer phyllosilicates possessing a lattice charge efficiency in the range of 0.2 to 0.4g equivalent per half unit cell as described in EP 350 288 (Unilever). Also encompassed in the present invention are any polymeric lubricant suitable for softening a fabric. These include silicone and in particular those described in GB1,549,180; EP 459 821 (Unilever) and EP 459 822 (Unilever).

When encompassed in the present invention, these improved fabric softening entities will be generally comprised at a level of from 0.5% to 50%, preferably from 1% to 30%, more preferably from 2% to 15% by weight of the total composition.

***Dye fixative agents***

Further suitable chemical entities suitable for the present invention are dye fixative entities wherein at least one benefit agent is a dye fixative agent, linked to a Mimic CBD. It has been surprisingly found that such dye fixative entities in the laundry detergent and/or fabric care compositions of the present invention, provide enchanced anti-wear properties and colour appearance due to very effective deposition of the dye fixative to the fabric.

Dye fixative agents are well-known, commercially available materials which are designed to improve the appearance of dyed fabric by minimising the loss of dye from fabrics due to washing. Many dye fixatives are cationic, and are based on various quatemized or otherwise cationically charged organic nitrogen compounds. Fixatives are available under various trade names from several suppliers. Representative examples include: CROSCOLOR PMF (July 1981, Code No. 7894) and CROSCOLOR NOFF (January 1988, Code No. 8544) from Crosfield; INDOSOL E-50 (February 27, 1984, Ref. No. 6008.35.84; polyethyleneamine-based) from Sandoz; SANDOFIX TPS, which is also available from Sandoz and is a preferred polycationic fixative for use herein and SANDOFIX SWE (cationic resinous compound), REWIN SRF, REWIN SRF-O and REWIN DWR from CHT-Beitlich GMBH. Other cationic dye fixative agents are described in "Aftertreatments for improving the fastness of dyes on textile fibres" by Christopher C. Cook (REV. PROG. COLORATION Vol. 12,1982).

Other dye fixative agents suitable for use in the present invention are ammonium compounds such as fatty acid - diamine condensates e.g. the hydrochloride, acetate, metosulphate and benzyl hydrochloride of oleyldiethyl aminoethylamide, oleylmethyl-diethylenediaminemethsulphate, monostearyl-ethylene diaminotrimethylammonium methosulphate and oxidised products of tertiary amines; derivatives of polymeric alkyldiamines, polyamine-cyanuric chloride condensates and aminated glycerol dichlorohydrins.

When encompassed in the compositions of the present invention, these dye fixative entities are generally comprised at a level of from 0.01% to 30%, preferably from 0.1% to 15% by weight of the total composition.

***Soil release agents***

A further suitable chemical entity for the compositions of the present invention is a soil release entity wherein at least one benefit agent is a soil release agent, linked to a Mimic CBD. It has been surprisingly found that such soil release agents in the laundry detergent and/or fabric care compositions of the present invention, provide very effective deposition of the soil release agent onto the fabric and therefore better soil release performance.

Suitable soil release agents for use in the present invention include ethylene glycols oligomers, polyethylene glycol and derivatives such as transesterified polyethylene glycols, and propylene polyoxy ethylene. Other suitable soil release agents useful in compositions of the present invention are conventionally copolymers or terpolymers of terephthalic acid with ethylene glycol and/or propylene glycol units in various arrangements. Examples of such polymers are disclosed in the commonly assigned US Patent Nos. 4116885 and 4711730 and European Published Patent Application No. 0 272 033. A particular preferred polymer in accordance with EP-A-0 272 033 has the formula

$$(CH_3(PEG)_{43})_{0.75}(POH)_{0.25}[T\text{-}PO)_{2.8}(T\text{-}PEG)_{0.4}]T(PO\text{-}H)_{0.25}((PEG)_{43}CH_3)_{0.75}$$

where PEG is -$(OC_2H_4)O$-,PO is $(OC_3H_6O)$ and T is $(pcOC_6H_4CO)$.

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be end-capped by sulphobenzoate groups. However, some copolymers will be less than fully capped, and therefore their end groups may consist of monoester of ethylene glycol and/or propane 1-2 diol, thereof consist "secondarily" of such species.
The selected polyesters herein contain about 46% by weight of dimethyl terephthalic acid, about 16% by weight of propane -1.2 diol, about 10% by weight ethylene glycol about 13% by weight of dimethyl sulfobenzoic acid and about 15% by weight of sulfoisophthalic acid, and have a molecular weight of about 3.000. The polyesters and their method of preparation are described in detail in EPA 311 342.

Other soil release agents currently used in the detergent context are those of U.S. 4,968,451, November 6, 1990 to J.J. Scheibel and E.P. Gosselink: such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the nonionic end-capped 1,2-propylene/polyoxyethylene terephthalate polyesters of U.S. 4,711,730, December 8, 1987 to Gosselink et al, for example those produced by transesterification/oligomerization of poly(ethyleneglycol) methyl ether, DMT, PG and poly(ethyleneglycol) ("PEG"); the partly- and fully- anionic-end-capped oligomeric esters of U.S. 4,721,580, January 26, 1988 to Gosselink, such as oligomers from ethylene glycol ("EG"), PG, DMT and Na-3,6-dioxa-8-hydroxyoctanesulfonate; the nonionic-capped block polyester oligomeric compounds of U.S. 4,702,857, October 27, 1987 to Gosselink, for example produced from DMT, Me-capped PEG and EG and/or PG, or a combination of DMT, EG and/or PG, Me-capped PEG and Na-dimethyl-5-sulfoisophthalate; and the anionic, especially sulfoaroyl, end-capped terephthalate esters of U.S. 4,877,896, October 31, 1989 to Maldonado, Gosselink et al, the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in US 5,415,807.

When encompassed in the compositions of the present invention, these soil release entities are generally comprised at a level of from 0.01% to 20%, preferably from 0.1% to 5% by weight of the total composition.

***Brightener agents***

Also suitable for the purpose of the present invention, is a brightener entity wherein at least one benefit agent is a brightener, linked to a Mimic CBD. It has been found that such brightener entities within the laundry detergent and/or fabric care compositions of the present invention, provides better whiteness maintenance performance due to very effective deposition to the fabric.

Suitable brighteners for compositions of the present invention are:

- Carbocycles types of compounds such as distyrylbenzenes, distyrylbiphenyls and divinylstibenes,
- Triazinylaminostilbenes,
- Stilbenyl-2H-triazoles such as stilbenyl-2H-naphtol[1,2-d]triazoles and bis(1,2,3-triazol-2-yl)stilbenes,
- Benzoxazoles such as stilbenylbenzoxazoles and bis(benzoxazoles),
- Furans, Benzo[b]furnas and Benzimidazoles such as bis(benzo[b]furan-2-yl)biphenyls and cationic benzimidazoles,
- 1,3-Diphenyl-2-pyrazolines
- Coumarins
- Naphtalimides
- 1,3,5-triazin-2-yl-Derivatives.

The brightener agents, for example of the Coumarin type, can be attached to the NH2 group or to an amido group from the Mimic CBD and/or linking region.

Preferred are the bleach-stable brighteners such as 1,4-di(2-methylaminostyryl)benzene.

Other suitable brighteners are the hydrophilic optical brighteners having the structural formula:

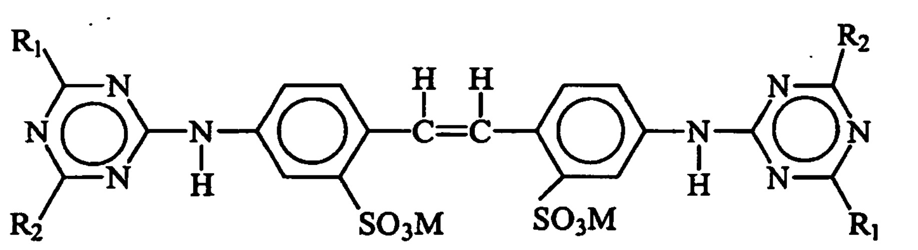

wherein $R_1$ is selected from anilino, N-2-bis-hydroxyethyl and NH-2-hydroxyethyl; $R_2$ is selected from N-2-bis-hydroxyethyl, N-2-hydroxyethyl-N-methylamino, morphilino, chloro and amino; and M is a salt-forming cation such as sodium or potassium.

- When in the above formula, $R_1$ is anilino, $R_2$ is N-2-bis-hydroxyethyl and M is a cation such as sodium, the brightener is 4,4',-bis[(4-anilino-6-(N-2-bis-hydroxyethyl)-s-triazine-2-yl)amino]-2,2'-stilbenedisulfonic acid and disodium salt. This particular brightener species is commercially marketed under the tradename Tinopal-UNPA-GX by Ciba-Geigy Corporation. Tinopal-UNPA-GX is the preferred hydrophilic optical brightener useful in the rinse added

compositions herein.

- When in the above formula, $R_1$ is anilino, $R_2$ is N-2-hydroxyethyl-N-2-methylamino and M is a cation such as sodium, the brightener is 4,4'-bis[(4-anilino-6-(N-2-hydroxyethyl-N-methylamino)-s-triazine-2-yl)amino]2,2'-stilbenedisulfonic acid disodium salt. This particular brightener species is commercially marketed under the tradename Tinopal 5BM-GX by Ciba-Geigy Corporation.
- When in the above formula, $R_1$ is anilino, $R_2$ is morphilino and M is a cation such as sodium, the brightener is 4,4'-bis[(4-anilino-6-morphilino-s-triazine-2-yl)amino]2,2'-stilbenedisulfonic acid, sodium salt. This particular brightener species is commercially marketed under the tradename Tinopal AMS-GX by Ciba Geigy Corporation.

Other suitable optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulphonate, monosodium 4',4" -bis-(2,4-dianilino-s-tri-azin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6- ylami-no)stilbene-2,2'disulphonate, sodium 2(stilbyl-4"-(naphtho-1',2':4,5)-1,2,3 - triazole-2"-sulphonate and 4,4'-bis (2-sulphostyryl)biphenyl. Highly preferred brighteners are the specific brighteners of copending European Patent application No. 95201943.8.

It has been surprisingly found that various brighteners not currently used in the detergent field could also be used in the present invention thanks to their increased fabric substantivity, in particular during a softening-through-the-wash process. Preferably, such brightener entities will not comprise a weak bond.

When encompassed in the compositions of the present invention, these brightnener entities will be generally comprised at a level from 0.001% to 10%, preferably from 0.005% to 3.5% by weight of the total composition.

***Other suitable benefit agents***

Also suitable as chemical entities for the compositions of the present invention are latex and resin entities wherein at least one benefit agent is a latex or resin agent, linked to a Mimic CBD. Latex is defined as a material suitable for refining the drape of fabric. Resins prevent the formation of pills on the fabrics.

Suitable latex materials include a polyvinylacetate homopolymer such as 9802 (Vinamul). Suitable resins are Knittex BE from Ciba-Geigy or silicas such as Crosanol NS from Crosfiled.

<u>The laundry detergent and/or fabric care compsoitions</u>

The present invention is further directed to laundry detergent and/or fabric care compositions comprising such chemical entity.

The laundry detergent and/or fabric care compositions of the invention will comprise at least one additional detergent and/or fabric care component. The precise nature of these additional components, and levels of incorporation thereof will depend on the physical form of the composition, and the nature of the cleaning operation for which it is to be used.

As already mentioned vide supra, all the benefit agents that may be linked to a Mimic CBD in accordance with the present invention, can also be incorporated into the laundry detergent and/or fabric care compositions of the present invention in their unmodified, conventional form.

The laundry detergent and/or fabric care compositions according to the invention can be liquid, paste, gels, bars, tablets, spray, foam, powder or granular forms. Granular compositions can also be in "compact" form, the liquid compositions can also be in a "concentrated" form.

The compositions of the invention may for example, be formulated as hand and machine laundry detergent compositions including laundry additive compositions and compositions suitable for use in the soaking and/or pretreatment of stained fabrics, rinse added fabric softener compositions. Pre-or post treatment of fabric include gel, spray and liquid fabric care compositions. A rinse cycle with or without the presence of softening agents is also contemplated.

When formulated as compositions suitable for use in a laundry machine washing method, the compositions of the invention preferably contain both a surfactant and a builder compound and additionally one or more detergent components preferably selected from organic polymeric compounds, bleaching agents, additional enzymes, suds suppressors, dispersants, lime-soap dispersants, soil suspension and antiredeposition agents and corrosion inhibitors. Laundry compositions can also contain softening agents, as additional detergent components.

The compositions of the invention can also be used as detergent additive products in solid or liquid form. Such additive products are intended to supplement or boost the performance of conventional detergent compositions and can be added at any stage of the cleaning process.

If needed the density of the laundry detergent compositions herein ranges from 400 to 1200 g/litre, preferably 600 to 950 g/litre of composition measured at 20°C.

The "compact" form of the compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition. In the compact compositions, the filler salt is present in amounts not exceeding 15% of the total composition, preferably not exceeding 10%, most preferably not exceeding 5% by weight of the composition. The inorganic filler salts, such as meant in the present compositions are selected from the alkali and alkaline-earth-metal salts of sulphates and chlorides. A preferred filler salt is sodium sulphate.

Liquid detergent compositions according to the present invention can also be in a "concentrated form", in such case, the liquid detergent compositions according the present invention will contain a lower amount of water, compared to conventional liquid detergents. Typically the water content of the concentrated liquid detergent is preferably less than 40%, more preferably less than 30%, most preferably less than 20% by weight of the detergent composition.

***Surfactant system***

The laundry detergent and/or fabric care compositions according to the present invention can comprise a surfactant system wherein the surfactant can be selected from nonionic and/or anionic and/or cationic and/or ampholytic and/or zwitterionic and/or semi-polar surfactants.

Surfactants are typically present at a level of from 0.1% to 60%, preferably from 1% to 35%, more preferably from 1% to 30% by weight of the total composition of the present invention.

Nonionic surfactants : Suitable nonionic surfactants for the surfactant systems of the present invention, are polyethylene, polypropylene, and polybutylene oxide condensates of alkyl phenols with the polyethylene oxide condensates being preferred. These compounds include the condensation products of alkyl phenols having an alkyl group containing from about 6 to about 14 carbon atoms, preferably from about 8 to about 14 carbon atoms, in either a straight-chain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with from about 1 to about 25 moles of ethylene oxide are suitable for use as the nonionic surfactant of the surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (the condensation product of $C_{11}$-$C_{15}$ linear alcohol with 9 moles ethylene oxide), Tergitol™ 24-L-6 NMW (the condensation product of $C_{12}$-$C_{14}$ primary alcohol with 6 moles ethylene oxide with a narrow molecular weight distribution), both marketed by Union Carbide Corporation; Neodol™ 45-9 (the condensation product of $C_{14}$-$C_{15}$ linear alcohol with 9 moles of ethylene oxide), Neodol™ 23-3 (the condensation product of $C_{12}$-$C_{13}$ linear alcohol with 3.0 moles of ethylene oxide), Neodol™ 45-7 (the condensation product of $C_{14}$-$C_{15}$ linear alcohol with 7 moles of ethylene oxide), Neodol™ 45-5 (the condensation product of $C_{14}$-$C_{15}$ linear alcohol with 5 moles of ethylene oxide) marketed by Shell Chemical Company, Kyro™ EOB (the condensation product of $C_{13}$-$C_{15}$ alcohol with 9 moles ethylene oxide), marketed by The Procter & Gamble Company, and Genapol LA O3O or O5O (the condensation product of $C_{12}$-$C_{14}$ alcohol with 3 or 5 moles of ethylene oxide) marketed by Hoechst. Preferred range of HLB in these products is from 8-11 and most preferred from 8-10.

Also useful as nonionic surfactants of the surfactant systems of the present invention are the alkylpolysaccharides disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e. g. a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units. The preferred alkylpolyglycosides have the formula

$$R^2O(C_nH_{2n}O)_t(glycosyl)_x$$

wherein $R^2$ is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominately the 2-position.

The condensation products of ethylene oxide with a hydrophobic base formed by the condensation of propylene oxide with propylene glycol are also suitable for use as nonionic surfactants for the present invention. The hydrophobic portion of these compounds will preferably have a molecular weight of from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially-available Plurafac™ LF404 and Pluronic™ surfactants, marketed by BASF.

Further suitable nonionic surfactants for the purposes of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic™ compounds, marketed by BASF.

Preferred for use as nonionic surfactants of the surfactant systems of the present invention are polyethylene oxide condensates of alkyl phenols, condensation products of primary and secondary aliphatic alcohols with from about 1 to about 25 moles of ethylene oxide, alkylpolysaccharides, and mixtures thereof. Most preferred are $C_8$-$C_{14}$ alkyl phenol ethoxylates having from 3 to 15 ethoxy groups and $C_8$-$C_{18}$ alcohol ethoxylates (preferably $C_{10}$ avg.) having from 2 to 10 ethoxy groups, and mixtures thereof.

Highly preferred nonionic surfactants are polyhydroxy fatty acid amide surfactants of the formula :

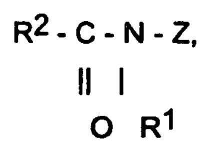

wherein $R^1$ is H, or $R^1$ is $C_{1-4}$ hydrocarbyl, 2-hydroxy ethyl, 2-hydroxy propyl or a mixture thereof, $R^2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is a straight $C_{11-15}$ alkyl or $C_{16-18}$ alkyl or alkenyl chain such as coconut alkyl or mixtures thereof, and Z is derived from a reducing sugar such as glucose, fructose, maltose, lactose, in a reductive amination reaction.

<u>Anionic surfactants</u> : Suitable anionic surfactants for use in the surfactant systems of the present invention, are linear alkyl benzene sulfonate, alkyl ester sulfonate surfactants including linear esters of $C_8$-$C_{20}$ carboxylic acids (i.e., fatty acids) which are sulfonated with gaseous $SO_3$ according to "The Journal of the American Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprise alkyl ester sulfonate surfactants of the structural formula:

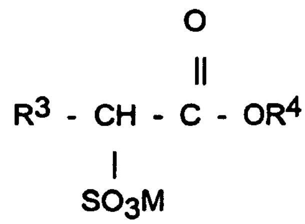

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as mono-ethanolamine, diethanolamine, and triethanolamine. Preferably, $R^3$ is $C_{10}$-$C_{16}$ alkyl, and $R^4$ is methyl, ethyl or isopropyl. Especially preferred are the methyl ester sulfonates wherein $R^3$ is $C_{10}$-$C_{16}$ alkyl.

Other suitable anionic surfactants suitable for the surfactant systems herein, include the alkyl sulfate surfactants which are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of $C_{12}$-$C_{16}$ are preferred for lower wash temperatures (e.g. below about 50°C) and $C_{16-18}$ alkyl chains are preferred for higher wash temperatures (e.g. above about 50°C).

Other anionic surfactants suitable for use in the surfactant systems of the present invention, include salts (including, for example, sodium, potassium, ammonium, and substituted ammonium salts such as mono-, di- and triethanolamine salts) of soap, $C_8$-$C_{22}$ primary of secondary alkanesulfonates, $C_8$-$C_{24}$ olefinsulfonates, sulfonated polycarboxylic acids prepared by sulfonation of the pyrolyzed product of alkaline earth metal citrates, e.g., as described in British patent specification No. 1,082,179, $C_8$-$C_{24}$ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated $C_{12}$-$C_{18}$ monoesters) and diesters of sulfosuccinates (especially saturated and unsaturated $C_6$-$C_{12}$ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula RO $(CH_2CH_2O)_k$-$CH_2COO$-M+ wherein R is a $C_8$-$C_{22}$ alkyl, k is an integer from 1 to 10, and M is a soluble salt-forming cation. Resin acids and hydrogenated resin acids are also suitable, such as rosin, hydrogenated rosin, and resin acids and hydrogenated resin acids present in or derived from tall oil.

Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).

When included therein, the laundry detergent compositions of the present invention typically comprise from about 1% to about 40%, preferably from about 3% to about 20% by weight of such anionic surfactants.

Highly preferred anionic surfactants include alkyl alkoxylated sulfate surfactants hereof are water soluble salts or acids of the formula $RO(A)_mSO3M$ wherein R is an unsubstituted $C_{10}$-$C_{24}$ alkyl or hydroxyalkyl group having a $C_{10}$-$C_{24}$ alkyl component, preferably a $C_{12}$-$C_{20}$ alkyl or hydroxyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethylammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are $C_{12}$-$C_{18}$ alkyl polyethoxylate (1.0) sulfate ($C_{12}$-$C_{18}E(1.0)M$), $C_{12}$-$C_{18}$ alkyl polyethoxylate (2.25) sulfate ($C_{12}$-$C_{18}E(2.25)M$), $C_{12}$-$C_{18}$ alkyl polyethoxylate (3.0) sulfate ($C_{12}$-$C_{18}E(3.0)M$), and $C_{12}$-$C_{18}$ alkyl polyethoxylate (4.0) sulfate ($C_{12}$-$C_{18}E(4.0)M$), wherein M is conveniently selected from sodium and potassium.

Further highly preferred anionic surfactants are the mid-chain-branched anionic surfactants described in the publshied applications by The Procter & Gamble Company: WO97/38957, WO97/39090, WO97/38956, WO97/39091, WO97/38972 and WO97/39091, hereby incorporated by reference. The compositions of the present invention generally

comprise from 0.1% to 50%, preferably from 0.5% to 40% more preferably from 1% to 35% by weight of the total composition of one or more longer alkyl chain, mid-chain branched surfactant compounds of the formula: $A^b$ - X - B wherein:

(I) $A^b$ is a hydrophobic mid-chain branched alkyl moiety, having in total 9 to 22 carbons in the moiety, preferably from 12 to about 18, having: (1) a longest linear carbon chain attached to the - X - B moiety in the range of from 8 to 21 carbon atoms; (2) one or more $C_1$ - $C_3$ alkyl moieties branching from this longest linear carbon chain; (3) at least one of the branching alkyl moieties is attached directly to a carbon of the longest linear carbon chain at a position within the range of the position 2 carbon, counting from position 1 carbon (#1) which is attached to the - X - B moiety, to the position of the terminal carbon minus 2 carbons, (the (ω - 2) carbon); and (4) when more than one of these compounds is present, the average total number of carbon atoms in the $A^b$-X moieties in the above formula is within the range of greater than 11 to 20, preferably 14.5 to about 18, more preferably from about 15 to about 17;

(II) B is a hydrophilic moiety selected from sulfates, sulfonates, amine oxides, polyoxyalkylene, preferably polyoxyethylene and polyoxypropylene, alkoxylated sulfates, polyhydroxy moieties, phosphate esters, glycerol sulfonates, polygluconates, polyphosphate esters, phosphonates, sulfosuccinates, sulfosuccaminates, polyalkoxylated carboxylates, glucamides, taurinates, sarcosinates, glycinates, isethionates, dialkanolamides, monoalkanolamides, monoalkanolamide sulfates, diglycolamides, diglycolamide sulfates, glycerol esters, glycerol ester sulfates, glycerol ethers, glycerol ether sulfates, polyglycerol ethers, polyglycerol ether sulfates, sorbitan esters, polyalkoxylated sorbitan esters, ammonioalkanesulfonates, amidopropyl betaines, alkylated quats, alkyated/polyhydroxyalkylated quats, alkylated quats, alkylated/polyhydroxylated oxypropyl quats, imidazolines, 2-yl-succinates, sulfonated alkyl esters, and sulfonated fatty acids; and

(III) X is selected from $-CH_2-$ and -C(O)-.

Preferred mid-banched anionic surfactants are of the above formula wherein the $A^b$ moiety is a branched primary alkyl moiety having the formula:

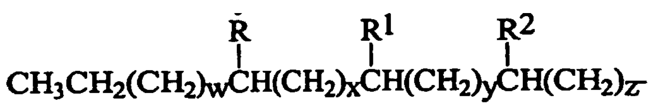

wherein the total number of carbon atoms in the branched primary alkyl moiety of this formula, including the R, $R^1$, and $R^3$ branching, is from 13 to 19; R, $R^1$, and $R^2$ are each independently selected from hydrogen and $C_1$-$C_3$ alkyl, preferably methyl, provided R, $R^1$, and $R^2$ are not all hydrogen and, when z is 0, at least R or $R^1$ is not hydrogen; w is an integer from 0 to 13; x is an integer from 0 to 13; y is an integer from 0 to 13; z is an integer from 0 to 13; and w + x + y + z is from 7 to 13.

More preferably, said surfactant has the formula:

(I) 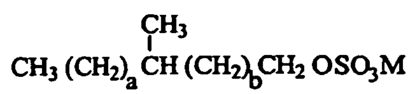 ,

or

..(II) 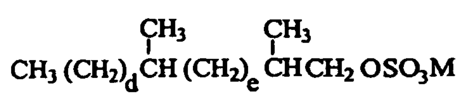 ,

or mixtures thereof; wherein M represents one or more cations; a, b, d, and e are integers, a+b is from 10 to 16, d+e is from 8 to 14 and wherein further

when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;

when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;

whereby, when more than one of these sulfate surfactants is present in the surfactant system, the average total number of carbon atoms in the branched primary alkyl moieties is from 11 to 20, preferably 14.5 to 18.
Particularly preferred is said surfactant which has an $A^b$ - X moiety comprising from 11 to 20, preferably 16 to 18 carbon atoms and B is a sulfate group.

Cationic surfactant : Suitable cationic surfactants suitable for detersive purposes herein, are those having one long-chain hydrocarbyl group. Examples of such cationic surfactants include the ammonium surfactants such as alkyltrimethylammonium halogenides, and those surfactants having the formula

$$[R^2(OR^3)_y][R^4(OR^3)_y]_2R^5N+X-$$

wherein $R^2$ is an alkyl or alkyl benzyl group having from about 8 to about 18 carbon atoms in the alkyl chain, each $R^3$ is selected from the group consisting of $-CH_2CH_2-$, - $CH_2CH(CH_3)-$, $-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl ring structures formed by joining the two $R^4$ groups, $-CH_2CHOH-CHOHCOR^6CHOHCH_2OH$
wherein $R^6$ is any hexose or hexose polymer having a molecular weight less than about 1000, and hydrogen when y is not 0; $R^5$ is the same as $R^4$ or is an alkyl chain wherein the total number of carbon atoms of $R^2$ plus $R^5$ is not more than about 18; each y is from 0 to about 10 and the sum of the y values is from 0 to about 15; and X is any compatible anion.

Quaternary ammonium surfactant suitable for the present invention has the formula (I):

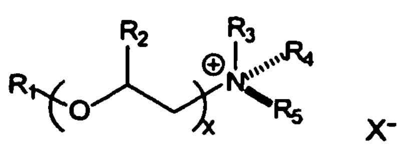

Formula I

whereby R1 is a short chainlength alkyl (C6-C10) or alkylamidoalkyl of the formula (II) :

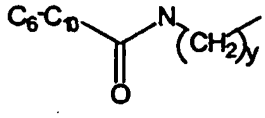

Formula II

y is 2-4, preferably 3.
whereby R2 is H or a C1-C3 alkyl,
whereby x is 0-4, preferably 0-2, most preferably 0,

whereby R3, R4 and R5 are either the same or different and can be either a short chain alkyl (C1-C3) or alkoxylated alkyl of the formula III,
whereby $X^-$ is a counterion, preferably a halide, e.g. chloride or methylsulfate.

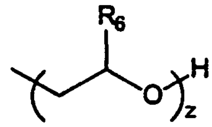

Formula III

R6 is $C_1$-$C_4$ and z is 1 or 2.
Preferred quaternary ammonium surfactants are those as defined in formula I whereby

$R_1$ is $C_8$, $C_{10}$ or mixtures thereof, x=o,
$R_3$, $R_4$ = $CH_3$ and $R_5$ = $CH_2CH_2OH$.

Highly preferred cationic surfactants are the water-soluble quaternary ammonium compounds useful in the present composition having the formula :

$$R_1R_2R_3R_4N^+X^- \qquad (i)$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and -$(C_2H_{40})_x$H where x has a value from 2 to 5, and X is an anion. Not more than one of $R_2$, $R_3$ or $R_4$ should be benzyl. The preferred alkyl chain length for $R_1$ is $C_{12}$-$C_{15}$ particularly where the alkyl group is a mixture of chain lengths derived from coconut or palm kernel fat or is derived synthetically by olefin build up or OXO alcohols synthesis. Preferred groups for $R_2R_3$ and $R_4$ are methyl and hydroxyethyl groups and the anion X may be selected from halide, methosulphate, acetate and phosphate ions.
Examples of suitable quaternary ammonium compounds of formulae (i) for use herein are :

coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
$C_{12-15}$ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl $(ethenoxy)_4$ ammonium chloride or bromide;
choline esters (compounds of formula (i) wherein $R_1$ is

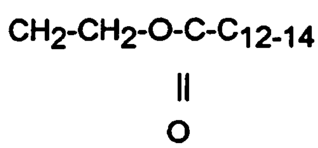

alkyl and $R_2R_3R_4$ are methyl).
di-alkyl imidazolines [compounds of formula (i)].

Other cationic surfactants useful herein are also described in U.S. Patent 4,228,044, Cambre, issued October 14, 1980 and in European Patent Application EP 000,224.
Typical fabric softening cationic surfactants include the water-insoluble quaternary-ammonium fabric softening actives or their corresponding amine precursor, the most commonly used having been di-long alkyl chain ammonium

chloride or methyl sulfate. Preferred cationic softeners among these include the following:

1) ditallow dimethylammonium chloride (DTDMAC);
2) dihydrogenated tallow dimethylammonium chloride;
3) dihydrogenated tallow dimethylammonium methylsulfate;
4) distearyl dimethylammonium chloride;
5) dioleyl dimethylammonium chloride;
6) dipalmityl hydroxyethyl methylammonium chloride;
7) stearyl benzyl dimethylammonium chloride;
8) tallow trimethylammonium chloride;
9) hydrogenated tallow trimethylammonium chloride;
10) $C_{12}$-$_{14}$ alkyl hydroxyethyl dimethylammonium chloride;
11) $C_{12-18}$ alkyl dihydroxyethyl methylammonium chloride;
12) di(stearoyloxyethyl) dimethylammonium chloride (DSOEDMAC);
13) di(tallow-oxy-ethyl) dimethylammonium chloride;
14) ditallow imidazolinium methylsulfate;
15) 1-(2-tallowylamidoethyl)-2-tallowyl imidazolinium methylsulfate.

Biodegradable quaternary ammonium compounds have been presented as altematives to the traditionally used di-long alkyl chain ammonium chlorides and methyl sulfates. Such quaternary ammonium compounds contain long chain alk(en)yl groups interrupted by functional groups such as carboxy groups. Said materials and fabric softening compositions containing them are disclosed in numerous publications such as EP-A-0,040,562, and EP-A-0,239,910.

The quaternary ammonium compounds and amine precursors herein have the formula (I) or (II), below :

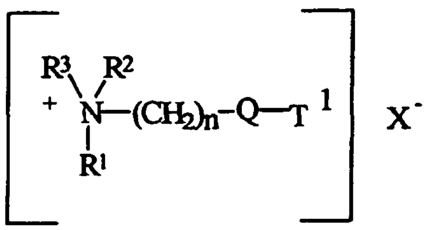

or

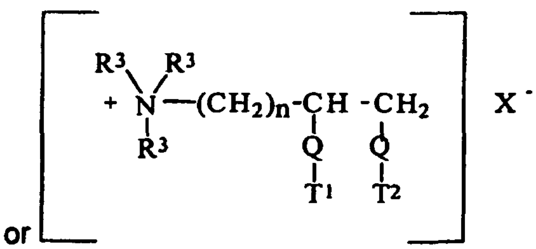

(I) (II)

wherein Q is selected from -O-C(O)-, -C(O)-O-, -O-C(O)-O-, $-NR^4-C(O)-$, $-C(O)-NR^4-$;
$R^1$ is $(CH_2)_n$-Q-$T^2$ or $T^3$;
$R^2$ is $(CH_2)_m$-Q-$T^4$ or $T^5$ or $R^3$;
$R^3$ is $C_1$-$C_4$ alkyl or $C_1$-$C_4$ hydroxyalkyl or H;
$R^4$ is H or $C_1$-$C_4$ alkyl or $C_1$-$C_4$ hydroxyalkyl;
$T^1$, $T^2$, $T^3$, $T^4$, $T^5$ are independently $C_{11}$-$C_{22}$ alkyl or alkenyl;
n and m are integers from 1 to 4; and
$X^-$ is a softener-compatible anion.

Non-limiting examples of softener-compatible anions indude chloride or methyl sulfate.

The alkyl, or alkenyl, chain $T^1$, $T^2$, $T^3$, $T^4$, $T^5$ must contain at least 11 carbon atoms, preferably at least 16 carbon atoms. The chain may be straight or branched.

Tallow is a convenient and inexpensive source of long chain alkyl and alkenyl material. The compounds wherein $T^1$, $T^2$, $T^3$, $T^4$, $T^5$ represents the mixture of long chain materials typical for tallow are particularly preferred.

Specific examples of quaternary ammonium compounds suitable for use in the aqueous fabric softening compositions herein include :

1) N,N-di(tallowyl-oxy-ethyl)-N,N-dimethyl ammonium chloride;
2) N,N-di(tallowyl-oxy-ethyl)-N-methyl, N-(2-hydroxyethyl) ammonium methyl sulfate;
3) N,N-di(2-tallowyl-oxy-2-oxo-ethyl)-N,N-dimethyl ammonium chloride;
4) N,N-di(2-tallowyl-oxy-ethylcarbonyl-oxy-ethyl)-N,N-dimethyl ammonium chloride;

5) N-(2-tallowyl-oxy-2-ethyl)-N-(2-tallowyl-oxy-2-oxo-ethyl)-N,N-dimethyl ammonium chloride;
6) N,N,N-tri(tallowyl-oxy-ethyl)-N-methyl ammonium chloride;
7) N-(2-tallowyl-oxy-2-oxo-ethyl)-N-(tallowyl-N,N-dimethyl-ammonium chloride; and
8) 1,2-ditallowyl-oxy-3-trimethylammoniopropane chloride;

and mixtures of any of the above materials.

The surfactant is preferably formulated to be compatible with enzyme components present in the composition. In liquid or gel compositions, the surfactant is most preferably formulated such that it promotes, or at least does not degrade, the stability of any enzyme in these compositions. The compositions of the present invention can further comprise ampholytic, zwitterionic and or semi-polar surfactants.

Ampholytic surfactants are suitable for use in the laundry detergent and/or fabric care compositions of the present invention can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate. See U.S. Patent No. 3,929,678 to Laughlin et al., issued December 30, 1975 at column 19, lines 18-35, for examples of ampholytic surfactants. When included therein, the laundry detergent and/or fabric care compositions of the present invention typically comprise from 0.2% to about 15%, preferably from about 1% to about 10% by weight of such ampholytic surfactants.

Zwitterionic surfactants suitable for use in the laundry detergent and/or fabric care compositions can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent No. 3,929,678 to Laughlin et al., issued December 30, 1975 at column 19, line 38 through column 22, line 48, for examples of zwitterionic surfactants.

When included therein, the laundry detergent and/or fabric care compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; water-soluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about 1 to about 3 carbon atoms. Semi-polar nonionic detergent surfactants include the amine oxide surfactants having the formula

$$\overset{0}{\uparrow}\; R^3(OR^4)xN(R^5)2$$

wherein $R^3$ is an alkyl, hydroxyalkyl, or alkyl phenyl group or mixtures therof containing from about 8 to about 22 carbon atoms; $R^4$ is an alkylene or hydroxyalkylene group containing from about 2 to about 3 carbon atoms or mixtures thereof; x is from 0 to about 3; and each $R^5$ is an alkyl or hydroxyalkyl group containing from about 1 to about 3 carbon atoms or a polyethylene oxide group containing from about 1 to about 3 ethylene oxide groups. The $R^5$ groups can be attached to each other, e.g., through an oxygen or nitrogen atom, to form a ring structure.

These amine oxide surfactants in particular include $C_{10}$-$C_{18}$ alkyl dimethyl amine oxides and $C_8$-$C_{12}$ alkoxy ethyl dihydroxy ethyl amine oxides.

When included therein, the compositions of the present invention typically comprise from 0.2% to about 15%, preferably from about 1% to about 10% by weight of such semi-polar nonionic surfactants.

Surfactants selected from the group of primary or tertiary amines also suitable for use in the laundry detergent and/or fabric care compositions of the present invention.

Suitable primary amines for use herein include amines according to the formula $R_1NH_2$ wherein $R_1$ is a $C_6$-$C_{12}$, preferably $C_6$-$C_{10}$ alkyl chain or $R_4X(CH_2)_n$, X is -O-,-C(O)NH- or -NH-, $R_4$ is a $C_6$-$C_{12}$ alkyl chain n is between 1 to 5, preferably 3. $R_1$ alkyl chains may be straight or branched and may be interrupted with up to 12, preferably less than 5 ethylene oxide moieties.

Preferred amines according to the formula herein above are n-alkyl amines. Suitable amines for use herein may be

selected from 1-hexylamine, 1-octylamine, 1-decylamine and laurylamine. Other preferred primary amines include C8-C10 oxypropylamine, octyloxypropylamine, 2-ethylhexyloxypropyl-amine, lauryl amido propylamine and amido propylamine.

Suitable tertiary amines for use herein include tertiary amines having the formula $R_1R_2R_3N$ wherein R1 and R2 are $C_1$-$C_8$ alkylchains or

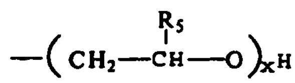

$R_3$ is either a $C_6$-$C_{12}$, preferably $C_6$-$C_{10}$ alkyl chain, or $R_3$ is $R_4X(CH_{2)n}$, whereby X is -O-, -C(O)NH- or -NH-, $R_4$ is a $C_4$-$C_{12,}$ n is between 1 to 5, preferably 2-3. $R_5$ is H or $C_1$-$C_2$ alkyl and x is between 1 to 6 .

$R_3$ and $R_4$ may be linear or branched ; $R_3$ alkyl chains may be interrupted with up to 12, preferably less than 5, ethylene oxide moieties.

Preferred tertiary amines are $R_1R_2R_3N$ where R1 is a C6-C12 alkyl chain, R2 and R3 are C1-C3 alkyl or

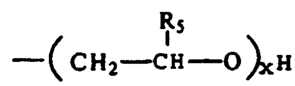

where R5 is H or CH3 and x = 1-2.

Also preferred are the amidoamines of the formula:

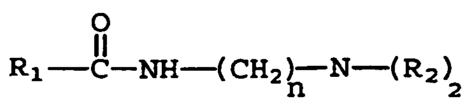

wherein $R_1$ is $C_6$-$C_{12}$ alkyl; n is 2-4,
preferably n is 3; $R_2$ and $R_3$ is $C_1$-$C_4$

Most preferred amines of the present invention include 1-octylamine, 1-hexylamine, 1-decylamine, 1-dodecylamine,C8-10oxypropylamine, N coco 1-3diaminopropane, coconutalkyldimethylamine, lauryldimethylamine, lauryl bis(hydroxyethyl)amine, coco bis(hydroxyehtyl)amine, lauryl amine 2 moles propoxylated, octyl amine 2 moles propoxylated, lauryl amidopropyl-dimethylamine, C8-10 amidopropyldimethylamine and C10 amidopropyl-dimethylamine. The most preferred amines for use in the compositions herein are 1-hexylamine, 1-octylamine, 1-decylamine, 1-dodecylamine. Especially desirable are n-dodecyldimethylamine and bishydroxyethylcoconutalkylamine and oleylamine 7 times ethoxylated, lauryl amido propylamine and cocoamido propylamine.

***Enzyme related ingredients***

Other suitable detergent ingredients that can be added are enzyme oxidation scavengers which are described in Copending European Patent application 92870018.6 filed on January 31, 1992. Examples of such enzyme oxidation scavengers are ethoxylated tetraethylene polyamines.

A range of enzyme materials and means for their incorporation into synthetic detergent compositions is also disclosed in WO 9307263 A and WO 9307260 A to Genencor International, WO 8908694 A to Novo, and U.S. 3,553,139, January 5, 1971 to McCarty et al. Enzymes are further disclosed in U.S. 4,101,457, Place et al, July 18, 1978, and in U.S. 4,507,219, Hughes, March 26, 1985. Enzyme materials useful for liquid detergent formulations, and their incorporation into such formulations, are disclosed in U.S. 4,261,868, Hora et al, April 14, 1981. Enzymes for use in detergents can be stabilised by various techniques. Enzyme stabilisation techniques are disclosed and exemplified in U.S. 3,600,319, August 17, 1971, Gedge et al, EP 199,405 and EP 200,586, October 29, 1986, Venegas. Enzyme stabilisation systems are also described, for example, in U.S. 3,519,570. A useful Bacillus, sp. AC13 giving proteases, xylanases and cellulases, is described in WO 9401532 A to Novo.

***Builder system***

The laundry detergent and/or fabric care compositions according to the present invention may further comprise a builder system. Any conventional builder system is suitable for use herein including aluminosilicate materials, silicates, polycarboxylates, alkyl- or alkenyl-succinic acid and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid. Phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.
Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates containing one carboxy group include lactic acid, glycolic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlegenschrift 2,446,686, and 2,446,687 and U.S. Patent No. 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.
Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis,cis-tetracarboxylates, cyclopentadienide pentacarboxylates, 2,3,4,5-tetrahydro-furan - cis, cis, cis-tetracarboxylates, 2,5-tetrahydro-furan -cis - dicarboxylates, 2,2,5,5-tetrahydrofuran - tetracarboxylates, 1,2,3,4,5,6-hexane -hexa-carboxylates and and carboxymethyl derivatives of polyhydric alcohols such as sorbitol, mannitol and xylitol. Aromatic poly-carboxylates include mellitic acid, pyromellitic acid and the phthalic acid derivatives disclosed in British Patent No. 1,425,343.
Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid. More preferred builders are zeolite A and sodium tripolyphosphate. Preferred builder systems for use in liquid detergent compositions of the present invention are soaps and polycarboxylates.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated from each other by not more than two carbon atoms.

Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in the compositions of the present invention at a level of from 5% to 80%, preferably from 10% to 70%, more preferably from 30% to 60% by weight of the composition.

***Chelating Agents***

The laundry detergent and/or fabric care compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates.

Amino carboxylates useful as optional chelating agents include ethylenediaminetetracetates, N-hydroxyethylethylenediaminetriacetates, nitrilotriacetates, ethylenediamine tetraproprionates, triethylenetetraamine-hexacetates, diethylenetriaminepentaacetates, and ethanoldiglycines, alkali metal, ammonium, and substituted ammonium salts therein and mixtures therein.

Amino phosphonates are also suitable for use as chelating agents in the compositions of the present invention when at least low levels of total phosphorus are permitted in detergent compositions, and include ethylenediaminetetrakis (methylenephosphonates) as DEQUEST. Preferred, these amino phosphonates do not .contain alkyl or .alkenyl groups with more than about 6 carbon atoms.

Polyfunctionally-substituted aromatic chelating agents are also useful in the compositions herein. See U.S. Patent 3,812,044, issued May 21, 1974, to Connor et al. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer as described in U.S. Patent 4,704,233, November 3, 1987, to Hartman and Perkins.

The compositions herein may also contain water-soluble methyl glycine diacetic acid (MGDA) salts (or acid form) as a chelant or co-builder useful with, for example, insoluble builders such as zeolites, layered silicates and the like.

If utilized in the compositions of the present invention, these chelating agents will generally be comprised at a level of from 0.1% to 15%, preferably from 0.1% to 3.0% by weight of the composition.

***Suds suppressor***

Another optional ingredient for the laundry detergent and/or fabric care compositions of the present invention, are suds suppressor exemplified by silicones and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the suds suppressor is advantageously releasably incorporated in a water-soluble or water-dispersible, substantially non-surface-active detergent impermeable carrier. Alternatively the suds suppressor can be dissolved or dispersed in a liquid carrier and applied by spraying on to one or more of the other components.

A preferred silicone suds controlling agent is disclosed in Bartollota et al. U.S. Patent 3 933 672. Other particularly useful suds suppressors are the self-emulsifying silicone suds suppressors, described in German Patent Application DTOS 2 646 126 published April 28, 1977. An example of such a compound is DC-544, commercially available from Dow Coming, which is a siloxane-glycol copolymer. Especially preferred suds controlling agent are the suds suppressor system comprising a mixture of silicone oils and 2-alkyl-alcanols. Suitable 2-alkyl-alkanols are 2-butyl-octanol which are commercially available under the trade name Isofol 12 R. Such suds suppressor system are described in Copending European Patent application N 92870174.7 filed 10 November, 1992. Especially preferred silicone suds controlling agents are described in Copending European Patent application N°92201649.8. Said compositions can comprise a silicone/silica mixture in combination with fumed nonporous silica such as Aerosil$^{R}$.

The suds suppressors described above are normally employed in the compositions of the present invention, at levels of from 0.001% to 2%, preferably from 0.01% to 1% by weight of the composition.

***Dispersants***

Suitable dispersants for use in laundry detergent and/or fabric care compositions of the present invention are water-soluble organic salts such as homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 1,000 to 100,000. Especially, copolymer of acrylate and methylacrylate such as the 480N having a molecular weight of 4000, at a level from 0.5-20% by weight of composition can be added in the laundry detergent and/or fabric care compositions of the present invention.

Other suitable dispersants can be lime soap peptiser compounds, which have preferably a lime soap dispersing power (LSDP), as defined hereinafter of no more than 8, preferably no more than 7, most preferably no more than 6. The lime soap peptiser compound is preferably present at a level from 0.0001% to 20% by weight.

A numerical measure of the effectiveness of a lime soap peptiser is given by the lime soap dispersant power (LSDP) which is determined using the lime soap dispersant test as described in an article by H.C. Borghetty and C.A. Bergman, J. Am. Oil. Chem. Soc., volume 27, pages 88-90, (1950). This lime soap dispersion test method is widely used by practitioners in this art field being referred to, for example, in the following review articles; W.N. Linfield, Surfactant science Series, Volume 7, page 3; W.N. Linfield, Tenside surf. det., volume 27, pages 159-163, (1990); and M.K. Nagarajan, W.F. Masler, Cosmetics and Toiletries, volume 104, pages 71-73, (1989). The LSDP is the % weight ratio of dispersing agent to sodium oleate required to disperse the lime soap deposits formed by 0.025g of sodium oleate

in 30ml of water of 333ppm $CaCo_3$ (Ca:Mg=3:2) equivalent hardness.
Polymeric lime soap peptisers suitable for use herein are described in the article by M.K. Nagarajan, W.F. Masler, to be found in Cosmetics and Toiletries, volume 104, pages 71-73, (1989).

Hydrophobic bleaches such as 4-[N-octanoyl-6-aminohexanoyl]benzene sulfonate, 4-[N-nonanoyl-6-aminohexanoyl]benzene sulfonate, 4-[N-decanoyl-6-aminohexanoyl]benzene sulfonate and mixtures thereof; and nonanoyloxy benzene sulfonate together with hydrophilic / hydrophobic bleach formulations can also be used as lime soap peptisers compounds.
Surfactants may also be used as lime soap peptiser compounds, such as certain amine oxides, betaines, sulfobetaines, alkyl ethoxysulfates and ethoxylated alcohols. Exemplary surfactants having a LSDP of no more than 8 for use in accord with the present invention include $C_{16}$-$C_{18}$ dimethyl amine oxide, $C_{12}$-$C_{18}$ alkyl ethoxysulfates with an average degree of ethoxylation of from 1-5, particularly $C_{12}$-$C_{15}$ alkyl ethoxysulfate surfactant with a degree of ethoxylation of amount 3 (LSDP=4), and the $C_{14}$-$C_{15}$ ethoxylated alcohols with an average degree of ethoxylation of either 12 (LSDP=6) or 30, sold under the tradenames Lutensol A012 and Lutensol A030 respectively, by BASF GmbH.

***Dye transfer inhibitors (DTI)***

The laundry detergent and/or fabric care compositions of the present invention can also include compounds for inhibiting dye transfer from one fabric to another of solubilized and suspended dyes encountered during fabric laundering operations involving colored fabrics. These DTI compounds are generally comprised at a level of from 0.001% to 10 %, preferably from 0.01% to 2%, more preferably from 0.05% to 1% by weight of the total composition. The DTI compounds have the ability to complex or adsorb the fugitive dyes washed out of dyed fabrics before the dyes have the opportunity to become attached to other articles in the wash. Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof.

a) Polyamine N-oxide polymers :

The polyamine N-oxide polymers suitable for use contain units having the following structure formula:

(I) 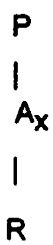

wherein P is a polymerisable unit, whereto the R-N-O group can be attached to or wherein the R-N-O group forms part of the polymerisable unit or a combination of both.
A is

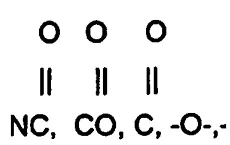

-S-, -N- ; x is O or 1 ;
R are aliphatic, ethoxylated aliphatics, aromatic, heterocyclic or alicyclic groups or any combination thereof whereto the nitrogen of the N-O group can be attached or wherein the nitrogen of the N-O group is part of these groups.
The N-O group can be represented by the following general structures :

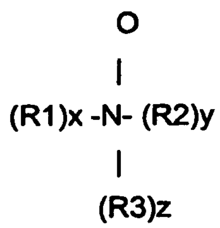

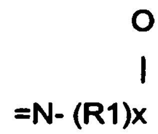

wherein R1, R2, and R3 are aliphatic groups, aromatic, heterocyclic or alicyclic groups or combinations thereof, x or/and y or/and z is 0 or 1 and wherein the nitrogen of the N-O group can be attached or wherein the nitrogen of the N-O group forms part of these groups.

The N-O group can be part of the polymerisable unit (P) or can be attached to the polymeric backbone or a combination of both.

Suitable polyamine N-oxides wherein the N-O group forms part of the polymerisable unit, comprise polyamine N-oxides wherein R is selected from aliphatic, aromatic, alicyclic or heterocyclic groups.

One class of said polyamine N-oxides comprises the group of polyamine N-oxides wherein the nitrogen of the N-O group forms part of the R-group. Preferred polyamine N-oxides are those wherein R is a heterocyclic group such as pyrridine, pyrrole, imidazole, pyrrolidine, piperidine, quinoline, acridine and derivatives thereof.

Another class of said polyamine N-oxides comprises the group of polyamine N-oxides wherein the nitrogen of the N-O group is attached to the R-group.

Other suitable polyamine N-oxides are the polyamine oxides whereto the N-O group is attached to the polymerisable unit.

Preferred class of these polyamine N-oxides are the polyamine N-oxides having the general formula (I) wherein R is an aromatic, heterocyclic or alicyclic groups wherein the nitrogen of the N-0 functional group is part of said R group.

Examples of these classes are polyamine oxides wherein R is a heterocyclic compound such as pyrridine, pyrrole, imidazole and derivatives thereof.

Another preferred class of polyamine N-oxides are the polyamine oxides having the general formula (I) wherein R are aromatic, heterocyclic or alicyclic groups wherein the nitrogen of the N-0 functional group is attached to said R groups.

Examples of these classes are polyamine oxides wherein R groups can be aromatic such as phenyl.

Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof.

The amine N-oxide polymers of the present invention typically have a ratio of amine to the amine N-oxide of 10:1 to 1:1000000. However the amount of amine oxide groups present in the polyamine oxide polymer can be varied by appropriate copolymerization or by appropriate degree of N-oxidation. Preferably, the ratio of amine to amine N-oxide is from 2:3 to 1:1000000. More preferably from 1:4 to 1:1000000, most preferably from 1:7 to 1:1000000. The polymers of the present invention actually encompass random or block copolymers where one monomer type is an amine N-oxide and the other monomer type is either an amine N-oxide or not.

The amine oxide unit of the polyamine N-oxides has a PKa < 10, preferably PKa < 7, more preferred PKa < 6.

The polyamine oxides can be obtained in almost any degree of polymerisation. The degree of polymerisation is not critical provided the material has the desired water-solubility and dye-suspending power.

Typically, the average molecular weight is within the range of 500 to 1000,000; preferably from 1,000 to 50,000, more preferably from 2,000 to 30,000, most preferably from 3,000 to 20,000.

b) Copolymers of N-vinylpyrrolidone and N-vinylimidazole :

The N-vinylimidazole N-vinylpyrrolidone polymers suitable for use in the compositions of the present invention, have an average molecular weight range from 5,000-1,000,000, preferably from 5,000-200,000.

Highly preferred polymers comprise a polymer selected from N-vinylimidazole N-vinylpyrrolidone copolymers wherein said polymer has an average molecular weight range from 5,000 to 50,000 more preferably from 8,000 to 30,000, most preferably from 10,000 to 20,000.

The average molecular weight range was determined by light scattering as described in Barth H.G. and Mays J.W. Chemical Analysis Vol 113,"Modern Methods of Polymer Characterization".

Highly preferred N-vinylimidazole N-vinylpyrrolidone copolymers have an average molecular weight range from 5,000 to 50,000; more preferably from 8,000 to 30,000; most preferably from 10,000 to 20,000.

The N-vinylimidazole N-vinylpyrrolidone copolymers characterized by having said average molecular weight range provide excellent dye transfer inhibiting properties while not adversely affecting the cleaning performance of laudnry detergent and/or fabric care compositions formulated therewith.
The N-vinylimidazole N-vinylpyrrolidone copolymer of the present invention has a molar ratio of N-vinylimidazole to N-vinylpyrrolidone from 1 to 0.2, more preferably from 0.8 to 0.3, most preferably from 0.6 to 0.4 .

c) Polyvinylpyrrolidone :

Suitable polyvinylpyrrolidones ("PVP") for use in the present invetnion, have an average molecular weight of from about 2,500 to about 400,000, preferably from about 5,000 to about 200,000, more preferably from about 5,000 to about 50,000, and most preferably from about 5,000 to about 15,000.
Suitable polyvinylpyrrolidones are commercially vailable from ISP Corporation, New York, NY and Montreal, Canada under the product names PVP K-15 (viscosity molecular weight of 10,000), PVP K-30 (average molecular weight of 40,000), PVP K-60 (average molecular weight of 160,000), and PVP K-90 (average molecular weight of 360,000). Other suitable polyvinylpyrrolidones which are commercially available from BASF Cooperation include Sokalan HP 165 and Sokalan HP 12; polyvinylpyrrolidones known to persons skilled in the detergent field (see for example EP-A-262,897 and EP-A-256,696).

d) Polyvinyloxazolidone :

Suitable polyvinyloxazolidones for use in the present invention, have an average molecular weight of from about 2,500 to about 400,000, preferably from about 5,000 to about 200,000, more preferably from about 5,000 to about 50,000, and most preferably from about 5,000 to about 15,000.

e) Polyvinylimidazole :

Suitable polyvinylimidazoles for use in the present invention, have an average of about 2,500 to about 400,000, preferably from about 5,000 to about 200,000, more preferably from about 5,000 to about 50,000, and most preferably from about 5,000 to about 15,000.

f) Cross-linked polymers :

Suitable for use in the compositions of the present invention are cross-linked polymers. Cross-linked polymers are polymers whose backbone are interconnected to a certain degree; these links can be of chemical or physical nature, possibly with active groups n the backbone or on branches; cross-linked polymers have been described in the Journal of Polymer Science, volume 22, pages 1035-1039.
In one embodiment, the cross-linked polymers are made in such a way that they form a three-dimensional rigid structure, which can entrap dyes in the pores formed by the three-dimensional structure. In another embodiment, the cross-linked polymers entrap the dyes by swelling.
Such cross-linked polymers are described in the co-pending patent application 94870213.9

***Colour care and fabric care benefits***

Other suitable technologies which provide a type of colour care benefit can also be included. Examples of these technologies are metallo catalysts for colour maintenance. Such metallo catalysts are described in copending European Patent Application No. 92870181.2. Dye fixing agents, polyolefin dispersion for anti-wrinkles and improved water absorbancy, perfume and amino-functional polymer for colour care treatment and perfume substantivity are further examples of colour care / fabric care technologies and are described in the co-pending Patent Application No. 96870140.9, filed November 07, 1996.
Other suitable fabric softening may be inorganic or organic in type. Inorganic softening agents are exemplified by the smectite clays disclosed in GB-A-1 400 898 and in USP 5,019,292. Organic fabric softening agents include the water insoluble tertiary amines as disclosed in GB-A1 514 276 and EP-B0 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146. Levels of smectite clay are normally in the range from 2% to 20%, more preferably from 5% to 15% by weight, with the material being added as a dry mixed component to the remainder of the formulation.
Organic fabric softening agents such as the water-insoluble tertiary amines or dilong chain amide materials

are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

***Bleaching agent***

Another detergent ingredient that can be included in the laundry detergent and/or fabric care compositions of the present invention is a bleaching agent.

The bleaching agent component for use herein can be any of the bleaching agents useful for cleaning compositions including oxygen bleaches as well as others known in the art. The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in U.S. Patent 4,483,781, U.S. Patent Application 740,446, European Patent Application 0,133,354 and U.S. Patent 4,412,934. Highly preferred bleaching agents also include 6-nonylamino-6-oxoperoxycaproic acid as described in U.S. Patent 4,634,551.

Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

Also suitable activators are acylated citrate esters such as disclosed in Copending European Patent Application No. 91870207.7.

Useful bleaching agents, including peroxyacids and bleaching systems comprising bleach activators and peroxygen bleaching compounds for use in the compositions of the present invention are described in our co-pending applications USSN 08/136,626, PCT/US95/07823, WO95/27772, WO95/27773, WO95/27774 and WO95/27775.

The hydrogen peroxide may also be present by adding an enzymatic system (i.e., an enzyme and a substrate therefore) which is capable of generating hydrogen peroxide at the beginning or during the washing and/or rinsing process. Such enzymatic systems are disclosed in EP Patent Application 91202655.6 filed October 9, 1991.

***Others***

Other components such as soil-suspending agents, soil-release agents, optical brighteners, abrasives, bactericides, tarnish inhibitors, and/or coloring agents may be employed.

Antiredeposition and soil suspension agents suitable herein include cellulose derivatives such as methylcellulose, carboxymethylcellulose and hydroxyethylcellulose, and homo-or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10%, preferably from 0.75% to 8%, more preferably from 1% to 6% by weight of the composition.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

It is well known in the art that free chlorine in tap water rapidly deactivates the enzymes comprised in detergent compositions. Therefore, using chlorine scavenger such as perborate, ammonium sulfate, sodium sulphite or polyethyleneimine at a level above 0.1% by weight of total composition, in the formulas will provide improved through the wash stability of the detergent enzymes. Compositions comprising chlorine scavenger are described in the European patent application 92870018.6 filed January 31, 1992.

Alkoxylated polycarboxylates such as those prepared from polyacrylates are useful herein to provide additional grease removal performance. Such materials are described in WO 91/08281 and PCT 90/01815 at p. 4 et seq., incorporated herein by reference. Chemically, these materials comprise polyacrylates having one ethoxy side-chain per every 7-8 acrylate units. The side-chains are of the formula $-(CH_2CH_2O)_m(CH_2)_nCH_3$ wherein m is 2-3 and n is 6-12. The side-chains are ester-linked to the polyacrylate "backbone" to provide a "comb" polymer type structure. The molecular weight can vary, but is typically in the range of about 2000 to about 50,000. Such alkoxylated polycarboxylates

can comprise from about 0.05% to about 10%, by weight, of the compositions herein.

**Method of washing**

The compositions of the invention may be used in essentially any washing, cleaning and/or fabric care methods, including soaking methods, pre-treatment methods, methods with rinsing steps for which a separate rinse aid composition may be added and post-treatment methods.

The process described herein comprises contacting fabrics with a laundering solution in the usual manner and exemplified hereunder. A conventional laundry method comprises treating soiled fabric with an aqueous liquid having dissolved or dispensed therein an effective amount of the laundry detergent and/or fabric care composition. The method of cleaning is preferably carried out at 5°C to 95°C, especially between 10°C and 60°C. The pH of the treatment solution is preferably from 7 to 12.

The following examples are meant to exemplify compositions of the present invention, but are not necessarily meant to limit or otherwise define the scope of the invention.

In the compositions, the enzymes levels are expressed by pure enzyme by weight of the total composition and unless otherwise specified, the detergent ingredients are expressed by weight of the total compositions. The abbreviated component identifications therein have the following meanings:

| | |
|---|---|
| LAS : | Sodium linear $C_{11-13}$ alkyl benzene sulphonate. |
| TAS : | Sodium tallow alkyl sulphate. |
| CxyAS : | Sodium $C_{1x}$ - $C_{1y}$ alkyl sulfate. |
| CxyEz : | $C_{1x}$ - $C_{1y}$ predominantly linear primary alcohol condensed with an average of z moles of ethylene oxide. |
| CxyEzS : | $C_{1x}$ - $C_{1y}$ sodium alkyl sulfate condensed with an average of z moles of ethylene oxide. |
| QAS 1 : | $R_2.N+(CH_3)_2(C_2H_4OH)$ with $R_2 = C_8-C_{11}$. |
| CFAA : | $C_{12}-C_{14}$ alkyl N-methyl glucamide. |
| TPKFA : | $C_{12}-C_{14}$ topped whole cut fatty acids. |
| Silicate : | Amorphous Sodium Silicate ($SiO_2:Na_2O$ ratio = 1.6-3.2). |
| Zeolite A : | Hydrated Sodium Aluminosilicate of formula $Na_{12}(A1O_2SiO_2)_{12}$. $27H_2O$ having a primary particle size in the range from 0.1 to 10 micromolareters (Weight expressed on an anhydrous basis). |
| Na-SKS-6 : | Crystalline layered silicate of formula $\delta$-$Na_2Si_2O_5$. |
| Citric : | Anhydrous citric acid. |
| Carbonate : | Anhydrous sodium carbonate with a particle size between 200 and 900 micromolaretres. |
| Sulphate : | Anhydrous sodium sulphate. |
| Mg Sulphate : | Anhydrous magnesium sulfate. |
| STPP : | Sodium tripolyphosphate. |
| TSPP : | Tetrasodium pyrophosphate. |
| MA/AA : | Random copolymer of 4:1 acrylate/maleate, average molecular weight about 70,000-80,000. |
| PB1 : | Anhydrous sodium perborate monohydrate of nominal formula $NaBO_2.H_2O_2$. |
| PB4 : | Sodium perborate tetrahydrate of nominal formula $NaBO_2.3H_2O.H_2O_2$. |
| Percarbonate : | Anhydrous sodium percarbonate of nominal formula $2Na_2CO_3.3H_2O_2$ . |
| TAED : | Tetraacetylethylenediamine. |
| DTPA : | Diethylene triamine pentaacetic acid. |
| HEDP : | 1,1-hydroxyethane diphosphonic acid. |
| DETPMP : | Diethyltriamine penta (methylene) phosphonate, marketed by Monsanto under the Trade name Dequest 2060. |
| EDDS : | Ethylenediamine-N,N'-disuccinic acid, (S,S) isomer in the form of its sodium salt |
| Photoactivated : Bleach | Sulfonated zinc phtalocyanine encapsulated in dextrin soluble polymer. |
| Protease : | Proteolytic enzyme sold under the tradename Savinase, Alcalase by Novo Nordisk A/S, the "protease D" variant with the substitution set N76D/S103A/V1041 and the protease described in PCT application Nos. PCT/US98/22588, PCT/US98/22482 and PCT/US98/22486 with the amino acid substitution set 101 G/103A/104I/159D/232V/236H/245R/248D/252K. |
| Amylase : | Amylolytic enzyme sold under the tradename Termamyl® and Duramyl® available from Novo Nordisk A/S and those variants having improved thermal stability with amino acid |

| | |
|---|---|
| | deletions R181· + G182· or T183· + G184· as described in WO95/35382. |
| Lipase : | Lipolytic enzyme sold under the tradename Lipolase, Lipolase Ultra by Novo Nordisk A/S and Lipomax by Gist-Brocades. |
| Cellulase : | Cellulytic enzyme sold under the tradename Carezyme, Celluzyme and/or Endolase by Novo Nordisk A/S. |
| Mimic-CBD-Endolase : | 1) Mimic CBD $(WE)_4K_2$ linked via a PEG linker to the cellulase sold under the tradename Endolase by Novo Nordisk A/S; as described in example 2; or<br>2) Mimic CBD $(WE)_4K_2$ linked via the Carezyme® linker to the cellulase sold under the tradename Endolase by Novo Nordisk A/S |
| CMC : | Sodium carboxymethyl cellulose. |
| PVP : | Polyvinyl polymer, with an average molecular weight of 60,000. |
| PVNO : | Polyvinylpyridine-N-Oxide, with an average molecular weight of 50,000. |
| PVPVI : | Copolymer of vinylimidazole and vinylpyrrolidone, with an average molecular weight of 20,000. |
| Brightener 1 : | Disodium 4,4'-bis(2-sulphostyryl)biphenyl. |
| Brightener 2 : | Disodium 4,4'-bis(4-anilino-6-morpholino-1.3.5-triazin-2-yl) stilbene-2:2'-disulfonate. |
| Silicone antifoam : | Polydimethylsiloxane foam controller with,siloxane-oxyalkylene copolymer as dispersing agent with a ratio of said foam controller to said dispersing agent of 10:1 to 100:1. |
| Suds Suppressor : | 12% Silicone/silica, 18% stearyl alcohol,70% starch in granular form. |
| SRP 1 : | Anionically end capped poly esters. |
| HMWPEO : | High molecular weight polyethylene oxide. |
| Mimic-CBD-Rotundial : | Mimic CBD (WE)4K2 with one or two Rotundial molecules. |
| Mimic-CBD- Damascone: | Mimic CBD (WE)4K15 linked with 15 Damascone molecules |

**Example 1**

According to the present invention, this example illustrates the coupling of a perfume citral, hygiene agent glutaraldehyde or insect control agent citronellal to $(WE)_4K_2$ Mimic CBD.

The reaction at equal mol takes place in mild conditions (pH 6 to 9.5, from 1 to 48hours /See for example Wirth, P at al 1991 Biorg.Chem 19, 133, 1991 and Chamow SM et al. Bioconjugate Chem. 4, 133 1994). The chemicals such as perfume (citral), insect control agent (citronellal) and hygiene agent (glutaraldehyde) are linked to NH2 groups of the Mimic CBD, linking region and/or polyreactive linking region via Schiff's base reaction.
The reaction could also be completed in anhydrous ethanol that contain a drying agent such as Sodium Sulphate.

As the Mimic CBD possesses 2 Lysine or in case there is a polyreactive linking region comprising more than one lysine, the reaction is identical with the appropriate number of chemicals per Mimic CBD. For example 2 equivalents of aldehyde will be added per mol of the $(WE)_4K_2$ Mimic CBD.

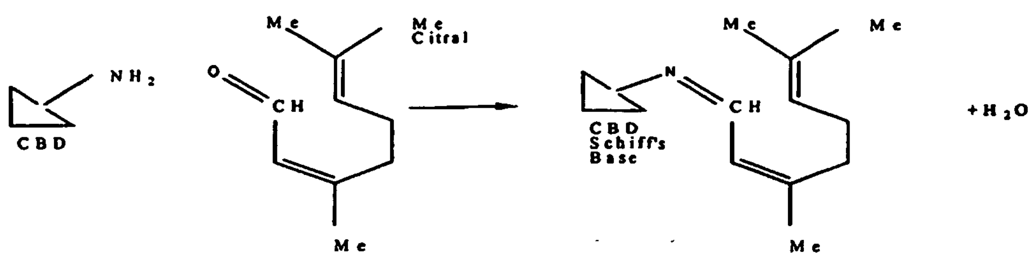

Release on dry fabrics is obtained by the slow hydrolysis of the Mimic CBD - perfume linkage.

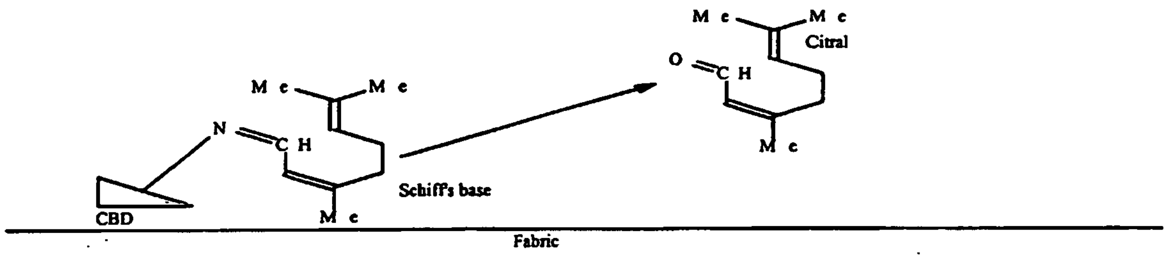

## Example 2

The following enzyme chemical entity : Mimic-CBD-PEG linker-Endolase was prepared according to the present invention.

1) A ratio of 1:8 of the Mimic CBD $(WE)_4K_2$ to Succinimydyl(acetylthio)acetate is incubated for 30 minutes at 37°C in a 50mM phosphate buffer at a pH7.5. to derivatizes the Mimic CBD with a protected linking group.

2) The reaction product obtained in step 1 is incubated for 2 hours at 20°C with 50mM phosphate buffer, 25mM EDTA and 0.5M of hydroxylamine.HCl at pH7.5. This step provides the activation of the protected linking group by deacetylation. The Mimic CBD is so prepared to be linked to the PEG linking region.

3) The cellulase sold under the tradename Endolase by Novo Nordisk A/S is linked with NS-PEG-VS in 50mM phosphate buffer, the molar ratio is 1:7 of Endolase to the PEG linker. The incubation time is 1 hour. This third step provides the linking of the Endolase cellulase to the linking region, with a reactive group on the linking region available to react with the derivatised Mimic CBD.

4) The conjugation of the products obtained in steps 2) and 3) is achieved as follows : Derivatised mimic CBD (step 1)) is reacted with derivatised Endolase (step 3)) in 50mM phosphate buffer in a molar ratio of 3:1 of Mimic CBD to Endolase, for 1 hour at room temperature.

## Example 3

The following high density laundry detergent compositions were prepared according to the present invention :

| | I | II |
|---|---|---|
| LAS | 6.0 | 6.0 |
| TAS | - | 0.1 |
| C25AS | 4.0 | 3.0 |
| C28AS | 1.0 | 2.0 |
| C25E5 | 4.6 | 4.0 |
| C25E3S | 5.0 | 4.5 |
| QAS 1 | 0.5 | 1.0 |
| Zeolite A | 20.0 | 20.0 |
| Citric | - | 2.5 |
| Carbonate | 10.0 | 13.0 |
| Na-SKS-6 | 10.0 | 10.0 |
| Silicate | 0.5 | 0.3 |
| Sulfate | - | 14 |
| Mg sulfate | - | 0.2 |
| MA/AA | 1.0 | 1.0 |
| CMC | 0.4 | 0.4 |
| Percarbonate | 18.0 | - |
| TAED | 3.9 | - |

(continued)

| | I | II |
|---|---|---|
| SRP 1 | - | 0.2 |
| EDDS | 0.5 | 0.5 |
| HEDP | 0.4 | 0.4 |
| Protease | 0.05 | 0.03 |
| Amylase | 0.008 | 0.008 |
| Cellulase | 0.0007 | 0.001 |
| Mimic-CBD-Endolase | - | 0.03 |
| Lipase | 0.01 | 0.01 |
| Photoactivated bleach (ppm) | 20 | 20 |
| PVNO/PVPVI | - | 0.1 |
| Brightener 1 | 0.09 | 0.09 |
| Perfume | 0.4 | 0.4 |
| Silicone antifoam | 0.3 | 0.3 |
| Mimic-CBD-Damascone | 1.0 | 0.2 |
| Density in g/litre | 850 | 850 |
| Miscellaneous and minors | Up to 100% | |

**Example 4**

The following granular laundry detergent compositions of particular utility under European machine wash conditions were prepared according to the present invention :

| | I | II |
|---|---|---|
| LAS | 7.0 | 6.0 |
| TAS | 1.0 | 1.0 |
| C24AS/C25AS | 1.0 | 1.0 |
| C25E3S | 0.1 | 0.1 |
| C25E5 | 4.0 | 4.0 |
| STPP | - | 20.0 |
| Zeolite A | 17.0 | - |
| Na-SKS-6 | 5.0 | 5.0 |
| Carbonate | 15.0 | 10.0 |
| Sulfate | 12.0 | 2.0 |
| MA/AA | 1.0 | 1.0 |
| CMC | 0.4 | 0.4 |
| PB4 | 15.0 | 15.0 |
| TAED | 2.5 | 2.5 |
| DETPMP | 0.2 | 0.2 |
| HEDP | 0.3 | 0.3 |
| Protease | 0.02 | 0.02 |
| Lipase | 0.004 | 0.004 |
| Cellulase | 0.0007 | 0.0007 |
| Amylase | 0.003 | 0.003 |
| PVP | 0.9 | 0.9 |
| Photoactivated bleach (ppm) | 20 | 20 |
| Brightener 1 | 0.15 | 0.15 |
| Clay | - | 10 |
| HMWPFO | - | 0.2 |
| Mimic-CBD-Damascone | 0.2 | 0.5 |

(continued)

| | I | II |
|---|---|---|
| Perfume | 0.3 | 0.3 |
| Silicone antifoam | 2.0 | 2.0 |
| Density in g/litre | 650 | 650 |
| Miscellaneous and minors | Up to 100% | |

**Example 5**

The following liquid detergent formulations were prepared according to the present invention (Levels are given in parts per weight) :

| | I | II |
|---|---|---|
| LAS | 11.5 | 7.0 |
| C45E2.25S | 4.0 | 4.0 |
| C23E7 | 4.0 | 3.0 |
| CFAA | 4.0 | 2.0 |
| TPKFA | 10.0 | 6.0 |
| Citric (50%) | 5.0 | 3.0 |
| $CaCl_2$ | 0.01 | 0.01 |
| Boric acid | 2.0 | 1.0 |
| Na hydroxide | 0.7 | 1.5 |
| Ethanol | 1.75 | 2.0 |
| 1,2 Propanediol | 9.0 | 8.0 |
| Monoethanolamine | 8.0 | 3.0 |
| Protease | 0.03 | 0.02 |
| Lipase | 0.002 | 0.001 |
| Amylase | 0.002 | 0.002 |
| Mimic-CBD-Endolase | 0.002 | 0.003 |
| SRP 1 | 0.2 | 0.1 |
| DTPA | 0.4 | 0.2 |
| PVNO | 0.4 | 0.2 |
| Brightener 1 | 0.2 | 0.1 |
| Silicone antifoam | 0.04 | 0.1 |
| CBD-Damascone | 1.0 | 0.2 |
| Miscellaneous and water | Up to 100% | |

**Example 6**

The following laundry bar detergent composition was prepared according to the present invention :

| | | | |
|---|---|---|---|
| LAS | 15.0 | SRP 1 | 0.3 |
| C45E7 | 2.0 | Amylase | 0.01 |
| Carbonate | 13.0 | Protease | 0.003 |
| Ca Carbonate | 30.0 | Cellulase | .0002 |
| Sulfate | 3.0 | Perfume | 0.3 |
| STPP | 10.0 | Mg sulfate | 3.0 |
| DETPMP | 0.6 | Mimic-CBD-Damascone | 1.0 |
| CMC | 1.0 | Mimic-CBD-Rotundial | 5.0 |
| Talc | 8.0 | Brightener 2 | 0.15 |
| Silicate | 4.0 | Photoactivated bleach (ppm) | 15.0 |

**Example 7**

The following granular fabric detergent compositions which provide "softening through the wash" capability were prepared according to the present invention :

| | I | II |
|---|---|---|
| C45AS | - | 10.0 |
| LAS | 7.6 | - |
| C68AS | 1.3 | - |
| C45E7 | 4.0 | - |
| C25E3 | - | 5.0 |
| Coco-alkyl-dimethyl hydroxy-ethyl ammonium chloride | 1.4 | 1.0 |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| PB1 | 15.0 | - |
| Percarbonate | - | 15.0 |
| TAED | 5.0 | 5.0 |
| Smectite clay | 10.0 | 10.0 |
| HMWPEO | - | 0.1 |
| Mimic-CBD-Damascone | 0.5 | 0.05 |
| Protease | 0.02 | 0.01 |
| Lipase | 0.02 | 0.01 |
| Amylase | 0.03 | 0.005 |
| Cellulase | 0.001 | - |
| Silicate | 3.0 | 5.0 |
| Carbonate | 10.0 | 10.0 |
| Suds suppressor | 1.0 | 4.0 |
| CMC | 0.2 | 0.1 |
| Miscellaneous and minors | Up to 100% | |

**Claims**

**1.** A mimic cellulose binding domain which is an amino acid sequence of less than 30 amino acids and is **characterised by** a protein deposition of more than 50%, preferably more than 75% on the cellulose structure (Avicel PH105) at a 1micromolar concentration, and by comprising at least 3 amino acids selected from the non-polar amino acids : glycine, alanine, proline, valine, leucine, isoleucine, tyrosine, tryptophan, phenylalanine and/or mixtures thereof.

**2.** A mimic cellulose binding domain according to claim 1 comprising at least 3 amino acids selected from the non-polar amino acids : valine, leucine, isoleucine, tyrosine, tryptophan, phenylalanine and/or mixtures thereof; preferably at least 3 non-polar amino acids selected from the aromatic amino acids : tyrosine, tryptophan or phenylalanine, and/or mixtures thereof.

**3.** A mimic cellulose binding domain according to claims 1-2 wherein the non-polar amino acids sequence are separated by at least 1, and preferably 1, separating amino acid.

**4.** A mimic cellulose binding domain according to claim 3 wherein said separating amino acids are selected from glutamine, asparagine, aspartate, glutamate, lysine, arginine, histidine and/or mixtures thereof.

5. A mimic cellulose binding domain according to claims 1-4 wherein the amino acid sequence has less than 12, preferably less than 10 amino acids.

6. A mimic cellulose binding domain according to claims 1-5 wherein said amino acid sequence further comprises at one or each end, one or more attachement amino acids selected from lysine, tyrosine, arginine, histidine, asparagine, glutamine, cysteine, and/or mixtures thereof, preferably from selected from lysine, cysteine and/or mixtures thereof.

7. A mimic celulose binding domain according to claim 6 wherein said attachment amino acids are added at a number of 1 to 20.

8. A cross-linked mimic cellulose binding domain entity formed by the linking of preferably 2 to 50, more preferably 2 to 10 mimic cellulose binding domains as defined in any of the preceding claims.

9. A chemical entity comprising a benefit agent linked to a mimic cellulose binding domain as defined in preceding claims.

10. A chemical entity according to claim 9 wherein said benefit agent is selected from enzymes, perfumes, hygiene agents, insect control agents, fabric softening agents, softening protein, soil release agents, bleaching agents, dye fixatives agents, brigtheners, latex, resins, and/or mixtures thereof, preferably from enzymes, perfumes, hygiene agents, insect control agents, and/or mixtures thereof.

11. A chemical entity according to claims 9-10 wherein said chemical component is linked to said mimic cellulose binding domain, via a linking region.

12. A cross-linked mimic cellulose binding domain entity according to claim 8 wherein said mimic cellulose binding domains are linked together via a linking region.

13. A chemical entity according to claim 11 or a cross-linked mimic cellulose binding domain entity according to claim 12, wherein said linking region is an amino acid linking region.

14. A chemical entity according to claim 11 or a cross-linked mimic cellulose binding domain entity according to claim 12, wherein said linking region is a polymer selected from PEG(NPC)2, (NH2)2-PEG, t-BOC-NH-PEG-NH2, MAL-PEG-NHS VS-PEG-NHS, polyethyleneimine polymers, polyvinvyl alcohol polymers, polyamines and/or mixtures thereof.

15. A chemical entity according to claims 11, 13 and 14 wherein said chemical component is linked to said mimic cellulose binding domain or to said linking region via a weak bond.

16. A laundry detergent and/or of fabric care compsoition comprising a cross-linked mimic celulose binding domain entity and/or a chemical entity, as defined in claims 8-15.

17. A composition according to claim 16 wherein said chemical entity is comprised at a level of from 0.00001% to 50%, preferably from 0.001% to 20%, more preferably from 0.1% to 10% by weight of the total composition.

18. A composition according to claim 16 wherein said a cross-linked mimic cellulose binding domain entity is comprised at a level of from 0.001% to 50%, preferably from 0.01% to 20%, more preferably from 0.1% to 10% by weight of the total composition.

19. A method of treating a fabric with a composition according to claims 16-18, for providing fabric deaning including soil and stain removal, dingy fabric cleaning and/or whiteness maintenance.

20. A method of treating a fabric with a composition according to claims 16-18, for providing fabric care including anti-wrinkling, anti-bobbling and anti-shrinkage properties and/or fabric softness.

**Patentansprüche**

1. Imitierte Cellulose Binding Domain, die eine Aminosäurensequenz von weniger als 30 Aminosäuren ist, **gekennzeichnet durch** eine Proteinabscheidung von mehr als 50 %, vorzugsweise mehr als 75 % der Cellulosestruktur (Avicel PH105) bei einer mikromolaren Konzentration von 1 und **dadurch**, dass sie zumindest 3 Aminosäuren umfasst, die ausgewählt sind aus den unpolaren Aminosäuren: Glycin, Alanin, Prolin, Valin, Leucin, Isoleucin, Tyrosin, Tryptophan, Phenylalanin und/oder Mischungen davon.

2. Imitierte Cellulose Binding Domain nach Anspruch 1, umfassend zumindest 3 Aminosäuren ausgewählt aus den unpolaren Aminosäuren: Valin, Leucin, Tyrosin, Tryptophan, Phenylalanin und/oder Mischungen davon; vorzugsweise zumindest 3 unpolaren Aminosäuren ausgewählt aus den aromatischen Aminosäuren: Tyrosin, Tryptophan oder Phenylalanin und/oder Mischungen davon.

3. Imitierte Cellulose Binding Domain nach einem der Ansprüche 1 oder 2, worin die unpolare Aminosäurensequenz durch zumindest 1 und vorzugsweise 1 Aminosäure separiert wird.

4. Imitierte Cellulose Binding Domain nach Anspruch 3, worin die separierenden Aminosäuren ausgewählt sind aus Glutamin, Asparagin, Aspartat, Glutamat, Lysin, Histidin und/oder Mischungen davon.

5. Imitierte Cellulose Binding Domain nach einem der Ansprüche 1 bis 4, worin die Aminosäurensequenz weniger als 12, vorzugsweise weniger als 10 Aminosäuren umfasst.

6. Imitierte Cellulose Binding Domain nach einem der Ansprüche 1 bis 5, worin die Sequenz von Aminosäuren an einem oder jedem Ende zusätzlich eine oder mehrere Anlagerungsaminosäuren ausgewählt aus Lysin, Tyrosin, Arginin, Histidin, Asparigin, Glutamin, Cystein und/oder Mischungen davon, vorzugsweise ausgewählt aus einer Auswahl von Lysin, Cystein und/oder Mischungen davon.

7. Imitierte Cellulose Binding Domain nach Anspruch 6, worin die Anlagerungsaminosäuren in einer Anzahl von 1 bis 20 hinzugefügt werden.

8. Vernetzte imitierte Cellulose Binding Domaineinheit gebildet durch Vernetzen von vorzugsweise 2 bis 50, mehr bevorzugt 2 bis 10 imitierten Cellulose Binding Domains gemäß Bestimmung in einem der vorhergehenden Ansprüche.

9. Chemische Einheit umfassend einen mit einer imitierten Cellulose Binding Domain gemäß Bestimmung in einem der vorhergehenden Ansprüche vernetzten Wirkstoff.

10. Chemische Einheit nach Anspruch 9, worin der Wirkstoff ausgewählt ist aus Enzymen, Duftstoffen, Hygienemitteln, Insektenbekämpfungsmitteln, Textilweichmachern, Weichmacherprotein, Schmutzabweisemitteln, Bleichmitteln, Farbfixiermitteln, Aufhellern, Latex, Harzen, und/oder Mischungen davon, vorzugsweise aus Enzymen, Duftstoffen, Hygienemitteln, Insektenbekämpfungsmitteln und/oder Mischungen davon.

11. Chemische Einheit nach Anspruch 9 oder 10, worin der chemische Bestandteil über einen Vernetzungsbereich mit der imitierten Cellulose Binding Domain vemetzt ist.

12. Vemetzte imitierte Cellulose Binding Domaineinheit nach Anspruch 8, worin die Imitierte Cellulose Binding Domaineinheiten über einen Vernetzungsbereich miteinander vemetzt sind.

13. Chemische Einheit nach Anspruch 11 oder vernetzte imitierte Cellulose Binding Domaineinheit nach Anspruch 12, worin der Vernetzungsbereich ein Aminosäurenvernetzungsbereich ist.

14. Chemische Einheit nach Anspruch 11 oder vernetzte imitierte Cellulose Binding Domaineinheit nach Anspruch 12, worin der Vernetzungsbereich ein Polymer ausgewählt aus PEG(NPC)2, (NH2)2-PEG, t-BOC-NH-PEG-NH2, MAL-PEG-NHS VS-PEG-NHS, Polyethylenimin Polymeren, Polyvinylalkoholpolymeren, Polyaminen und/oder Mischungen davon ist.

15. Chemische Einheit nach Anspruch 11, 13 und 14, worin der chemische Bestandteil über eine weiche Bindung mit der imitierten Cellulose Binding Domain oder mit dem Vernetzungsbereich vernetzt ist.

**16.** Wäschewaschmittel und/oder Textilpflegezusammensetzung, umfassend eine vernetzte imitierte Cellulose Binding Domaineinheit und/oder eine chemische Einheit gemäß Bestimmung in den Ansprüchen 8 bis 15.

**17.** Zusammensetzung nach Anspruch 16, worin die chemische Einheit auf einem Niveau von 0,00001 Gew.-% bis 50 Gew.-%, vorzugsweise von 0,001 Gew.% bis zu 20 Gew.-% und am meisten bevorzugt von 0,1 Gew.-% bis 10 Gew.-% bezogen auf die Gesamtzusammensetzung enthalten ist.

**18.** Zusammensetzung nach Anspruch 16, worin die vernetzte imitierte Cellulose Binding Domaineinheit auf einem Niveau von 0,001 Gew.-% bis 50 Gew.-%, vorzugsweise von 0,01 Gew.-% bis 20 Gew.-% und mehr bevorzugt von 0,01 Gew.-% bis zu 10 Gew.-% bezogen auf die Gesamtzusammensetzung enthalten ist.

**19.** Verfahren zur Behandlung eines Gewebes mit einer Zusammensetzung nach den Ansprüchen 16 bis 18 zur Bereitstellung von Gewebereinigung einschließlich Schmutz- und Fleckenbeseitigung, Reinigung von vergrautem Gewebe und/oder Aufrechterhaltung von Weiße.

**20.** Verfahren zur Behandlung eines Gewebes mit einer Zusammensetzung nach den Ansprüchen 16 bis 18 zur Bereitstellung von Textilpflege einschließlich Anti-Knitterbehandlung, Anti-Flusenbehandlung und/oder Gewebeweichmachung.

**Revendications**

**1.** Domaine mimétique de liaison à la cellulose qui est une séquence aminoacide de moins de 30 acides aminés et est **caractérisé par** un dépôt protéique de plus de 50 %, de préférence de plus de 75 % sur la structure cellulosique (Avicel PH105) à une concentration micromolaire de 1, et comprenant au moins 3 acides aminés choisis parmi les acides aminés apolaires: glycine, alanine, proline, valine, leucine, isoleucine, tyrosine, tryptophane, phénylalanine et/ou leurs mélanges.

**2.** Domaine mimétique de liaison à la cellulose selon la revendication 1 comprenant au moins 3 acides aminés choisis parmi les acides aminés apolaires: valine, leucine, isoleucine, tyrosine, tryptophane, phénylalanine et/ou leurs mélanges; de préférence au moins 3 acides aminés apolaires choisis parmi les acides aminés aromatiques: tyrosine, tryptophane ou phénylalanine, et/ou leurs mélanges.

**3.** Domaine mimétique de liaison à la cellulose selon les revendications 1 et 2 dans lequel les séquences d'acides aminés apolaires sont séparées par au moins 1, et de préférence 1, acide aminé de séparation.

**4.** Domaine mimétique de liaison à la cellulose selon la revendication 3 dans lequel lesdits acides aminés de séparation sont choisis parmi la glutamine, l'asparagine, l'aspartate, le glutamate, la lysine, l'arginine, l'histidine et/ou leurs mélanges.

**5.** Domaine mimétique de liaison à la cellulose selon les revendications 1 à 4 dans lequel la séquence aminoacide a moins de 12, de préférence moins de 10 acides aminés.

**6.** Domaine mimétique de liaison à la cellulose selon les revendications 1 à 5 dans lequel ladite séquence aminoacide comprend en outre à une ou chaque extrémité, un ou plusieurs acides aminés d'attachement choisis parmi la lysine, la tyrosine, l'arginine, l'histidine, l'asparagine, la glutamine, la cystéine, et/ou leurs mélanges, de préférence choisis parmi la lysine, la cystéine et/ou leurs mélanges.

**7.** Domaine mimétique de liaison à la cellulose selon la revendication 6 dans lequel lesdits acides aminés d'attachement sont ajoutés à un nombre de 1 à 20.

**8.** Entité du domaine mimétique de liaison à la cellulose réticulée formée par la liaison de de préférence 2 à 50, plus préférablement 2 à 10 domaines mimétiques de liaison à la cellulose tels que définis dans l'une quelconque des revendications précédentes.

**9.** Entité chimique comprenant un agent bénéfique lié à un domaine mimétique de liaison à la cellulose tel que défini dans les revendications précédentes.

**10.** Entité chimique selon la revendication 9 dans laquelle ledit agent bénéfique est choisi parmi des enzymes, des parfums, des agents hygiéniques, des agents insecticides, des agents d'adoucissement des tissus, une protéine adoucissante, des agents de libération des salissures, des agents de blanchiment, des agents fixateurs de colorants, des azurants, un latex, des résines, et/ou leurs mélanges, de préférence parmi des enzymes, des parfums, des agents hygiéniques, des agents insecticides, et/ou leurs mélanges.

**11.** Entité chimique selon les revendications 9 à 10 dans laquelle ledit composant chimique est lié au dit domaine mimétique de liaison à la cellulose, par une région de liaison.

**12.** Entité du domaine mimétique de liaison à la cellulose réticulée selon la revendication 8 dans laquelle lesdits domaines mimétiques de liaison à la cellulose sont liés ensemble par une région de liaison.

**13.** Entité chimique selon la revendication 11 ou entité du domaine mimétique de liaison à la cellulose réticulée selon la revendication 12, dans laquelle ladite région de liaison est une région de liaison aminoacide.

**14.** Entité chimique selon la revendication 11 ou entité du domaine mimétique de liaison à la cellulose réticulée selon la revendication 12, dans laquelle ladite région de liaison est un polymère choisi parmi PEG(NPC)2, (NH2)2-PEG, t-BOC-NH-PEG-NH2, MAL-PEG-NHS VS-PEG-NHS, des polymères de polyéthylène-imine, des polymères d' alcool polyvinylique, des polyamines et/ou leurs mélanges.

**15.** Entité chimique selon les revendications 11, 13 et 14 dans laquelle ledit composant chimique est lié au dit domaine mimétique de liaison à la cellulose ou à ladite région de liaison par une liaison faible.

**16.** Détergent pour le lavage du linge et/ou composition pour le soin des tissus comprenant une entité du domaine mimétique de liaison à la cellulose réticulée et/ou une entité chimique, telles que définies dans les revendications 8 à 15.

**17.** Composition selon la revendication 16 dans laquelle ladite entité chimique est comprise à un niveau allant de 0,00001 % à 50 %, de préférence de 0,001 % à 20 %, plus préférablement de 0,1 % à 10 % en poids de la composition totale.

**18.** Composition selon la revendication 16 dans laquelle ladite entité du domaine mimétique de liaison à la cellulose réticulée est comprise à un niveau allant de 0,001 % à 50 %, de préférence de 0,01 % à 20 %, plus préférablement de 0,1 % à 10 % en poids de la composition totale.

**19.** Procédé de traitement d'un tissu avec une composition selon les revendications 16 à 18, pour apporter un nettoyage des tissus y compris une élimination des salissures et des taches, un nettoyage des tissus ternes et/ou un maintien de la blancheur.

**20.** Procédé de traitement d'un tissu avec une composition selon les revendications 16 à 18, pour apporter un soin des tissus y compris des propriétés d'anti-froissage, d'anti-boulochage, d'anti-rétrécissement et/ou de douceur des tissus.